# EUROPEAN PATENT APPLICATION

(11) **EP 4 130 033 A1**
(43) Date of publication of application: **08.02.2023**
(21) Application number: 21781490.4
(22) Date of filing: 29.03.2021
(51) Int. Cl.: C07K 16/00, C07K 16/28, C07K 16/46, C40B 40/10, C12N 15/13

(54) **EPITOPE REGION-BRIDGING BIPARATOPIC ANTIBODY AND METHOD FOR PRODUCING SAME**

(30) Priority: 30.03.2020 JP 2020061014
(71) Applicant: National Institutes of Biomedical Innovation, Health and Nutrition, Ibaraki-shi, Osaka 567-0085 (JP)
(72) Inventor: AKIBA, Hiroki, Osaka 567-0085 (JP); TSUMOTO, Kouhei, Osaka 567-0085 (JP); NAGATA, Satoshi, Osaka 567-0085 (JP)
(74) Representative: Engelhard, Markus
(86) International application number: PCT/JP2021/013341
(87) International publication number: WO 2021/200840

(57) **Abstract**

The present disclosure provides a technique for designing an epitope region-bridging biparatopic antibody, and also provides, through this technique, an antibody that has an improved agonist or antagonist function for an antigen or that promotes or enhances a chemical reaction catalyzed by an enzyme serving as an antigen. Provided is an epitope region-bridging biparatopic antibody, an antigen-binding fragment thereof or a functional equivalent of the same that has antigen specificities to a first epitope belonging to a first epitope group, among a plurality of epitope region groups in an antigen, and to a second epitope belonging to a second epitope group contained in an epitope region different from the epitope region in which the first epitope group is contained, said antibody comprising the sequences of a heavy chain variable region and a light chain variable region derived from an antibody against the first epitope and the sequences of a heavy chain variable region and a light chain variable region derived from an antibody against the second epitope.

## Description

### [Technical Field]

The present disclosure relates to an epitope region-bridging biparatopic antibody and a method of manufacturing the same. More specifically, the present disclose relates to a biparatopic antibody with an improved function as an agonist or antagonist.

### [Background Art]

In recent years, drugs using an antibody have been studied as a very attractive molecularly targeted drug from the viewpoint of their target specificity. Antibody therapeutic drugs bind to a specific moiety (epitope) of a target. The usefulness of an antibody drug is not dependent on only the type of antigen to which an antibody binds. Perturbation of a protein function due to binding is dependent on where the interaction due to the bond occurs. Thus, the usefulness varies significantly depending on the epitope in an antigen. Therefore, an epitope region to which an antibody binds is a critical factor that determines the function of the antibody.

The inventors have so far developed an epitope normalized antibody panel, which is a technology for obtaining a panel of a minimum number of antibodies whose epitope regions are comprehensively and evenly distributed over the entire accessible surface of a target. An epitope normalized antibody panel is made by categorizing every structure on a target surface into epitope regions recognized by each group of antibodies based on a reactivity profile of a large number of antibodies that "comprehensively" recognize the structures. An epitope normalized antibody panel is obtained so that the antibody panel contains the least total number of antibodies comprising an group of antibodies to each of the epitope regions covering a target. Therefore, an epitope normalized antibody panel selects out epitope regions that are important for the expression of a function from thereamong (Patent Literature 1). This technology has reduced the labor required for identifying an epitope region to which an antibody binds, enabled investigation of functions associated with the epitope region from the early stages of development of antibody drugs, and contributed to the discovery and development of antibodies with various functions.

### [Citation List]

### [Patent Literature]

[PTL 1] International Publication No. WO 2018/092907

### [Summary of Invention]

### [Solution to Problem]

The present disclosure discovered an epitope region-bridging biparatopic antibody preparation technology that can further develop the epitope normalized antibody panel technology and enable creation of an antibody drug that maximizes the function/effect thereof. Specifically, the present disclosure developed a sophisticated technology for preparing an epitope region-bridging biparatopic antibody as a panel by further adding positional information of an epitope to the epitope normalized antibody panel technology, searching for a functional molecule, elucidating the functional mechanism thereof, and rationally designing an epitope region-bridging biparatopic antibody therethrough.

Therefore, the present disclosure provides the following.
(Item 1)
   An epitope region-bridging biparatopic antibody or an antigen binding fragment or functional equivalent thereof, comprising sequences of a heavy chain variable region and a light chain variable region derived from an antibody to a first epitope in an antigen that is activated by a target binding thereto, and sequences of a heavy chain variable region and a light chain variable region derived from an antibody to a second epitope that is different from the first epitope, wherein a complex having a structure with antigen: antibody of (m × n):n is formed by binding to the antigen, wherein n is the same number that is 1 or greater, and m is a maximum value of the number of antigen binding domains sharing a paratope.
(Item 2)
   The epitope region-bridging biparatopic antibody or an antigen binding fragment or functional equivalent thereof of item 1, wherein the first epitope belongs to a first epitope region selected from a plurality of epitope regions in the antigen, and the second epitope belongs to a second epitope region that is different from the first epitope region.
(Item 3)
   The epitope region-bridging biparatopic antibody or an antigen binding fragment or functional equivalent thereof of item 1 or 2, wherein m is 1.
(Item 4)
   The epitope region-bridging biparatopic antibody or an antigen binding fragment or functional equivalent thereof of any one of items 1 to 3, wherein a complex having an intramolecular bridged structure with antigen:antibody of 1:1 is formed by binding to the antigen.
(Item 5)
   The epitope region-bridging biparatopic antibody or an antigen binding fragment or functional equivalent thereof of any one of items 1 to 3, wherein a complex having an intermolecular bridged structure with antigen:antibody of n:n (where n is the same number that is 2 or greater) is formed by binding to the antigen.
(Item 6)
   The epitope region-bridging biparatopic antibody or an antigen binding fragment or functional equivalent thereof of any one of items 1 to 4, having antigen specificity to the first epitope and the second epitope that are present on the same side from each other on the antigen when binding to the antigen.
(Item 7)
   The epitope region-bridging biparatopic antibody or an antigen binding fragment or functional equivalent thereof of any one of items 1 to 3 and 5, having antigen specificity to the first epitope and the second epitope that are present on different sides from each other on the antigen when binding to the antigen.
(Item 8)
   The epitope region-bridging biparatopic antibody or an antigen binding fragment or functional equivalent thereof of any one of items 1 to 4 and 6, having antigen specificity to the first epitope and the second epitope that are present on a side that binds to the target molecule on the antigen when binding to the antigen.
(Item 9)
   The epitope region-bridging biparatopic antibody or an antigen binding fragment or functional equivalent thereof of any one of items 1 to 3, 5, and 7, having antigen specificity to the first epitope that is present on a side that binds to the target molecule on the antigen and the second epitope that is present on a side that does not bind to the target molecule on the antigen when binding to the antigen.
(Item 10)
   The epitope region-bridging biparatopic antibody or an antigen binding fragment or functional equivalent thereof of any one of items 1 to 4, 6, and 8, wherein antagonistic activity is enhanced relative to a naturally-occurring antibody.
(Item 11)
   The epitope region-bridging biparatopic antibody or an antigen binding fragment or functional equivalent thereof of any one of items 1 to 4, 6, 8, and 10, wherein agonistic activity is suppressed relative to a naturally-occurring antibody.
(Item 12)
   The epitope region-bridging biparatopic antibody or an antigen binding fragment or functional equivalent thereof of any one of items 1 to 11, wherein the antigen is a membrane protein.
(Item 13)
   The epitope region-bridging biparatopic antibody or an antigen binding fragment or functional equivalent thereof of any one of items 1 to 12, wherein the antigen is a membrane protein belonging to a TNF receptor super family.
(Item 14)
   The epitope region-bridging biparatopic antibody or an antigen binding fragment or functional equivalent thereof of any one of items 1 to 13, wherein the antigen is TNFR2.
(Item 15)
   An epitope region-bridging biparatopic antibody or an antigen binding fragment or functional equivalent thereof to TNFR2, comprising:
   (a) a heavy chain comprising CDR1, CDR2, and CDR3 of each of two heavy chain variable regions selected from the group consisting of SEQ ID NO: 1 (TR45 heavy chain), SEQ ID NO: 2 (TR92 heavy chain), SEQ ID NO: 3 (TR94 heavy chain), SEQ ID NO: 4 (TR96 heavy chain), and SEQ ID NO: 5 (TR109 heavy chain), or a functionally equivalent sequence thereof, or an amino acid sequence having sequence identity of at least 80% to said amino acid sequences; and
   (b) a light chain comprising CDR1, CDR2, and CDR3 of each of two light chain variable regions selected from the group consisting of SEQ ID NO: 6 (TR45 light chain), SEQ ID NO: 7 (TR92 light chain), SEQ ID NO: 8 (TR94 light chain), SEQ ID NO: 9 (TR96 light chain), and SEQ ID NO: 10 (TR109 light chain), wherein the light chain variable regions are derived from the same antibody as the antibody heavy chain of (a), or a functionally equivalent sequence thereof, or an amino acid sequence having sequence identity of at least 80% to said amino acid sequences.
(Item 16)
   The epitope region-bridging biparatopic antibody or an antigen binding fragment or functional equivalent thereof of item 15, comprising:
   (a) a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences; and
   (b) a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences.
(Item 17)
   The epitope region-bridging biparatopic antibody or an antigen binding fragment or functional equivalent thereof of item 15, which is:
   (i) an antibody comprising
      (a1) a heavy chain comprising CDR1, CDR2, and CDR3 of the amino acid sequence set forth in SEQ ID NO: 1 (TR45 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and
      (b1) a light chain comprising CDR1, CDR2, and CDR3 of the amino acid sequence set forth in SEQ ID NO: 6 (TR45 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences;
   (ii) an antibody comprising
      (a2) a heavy chain comprising CDR1, CDR2, and CDR3 of the amino acid sequence set forth in SEQ ID NO: 1 (TR45 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and
      (b2) a light chain comprising CDR1, CDR2, and CDR3 of the amino acid sequence set forth in SEQ ID NO: 6 (TR45 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences;
   (iii) an antibody comprising
      (a3) a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and
      (b3) a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences;
   (iv) an antibody comprising
      (a4) a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and
      (b4) a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences;
   (v) an antibody comprising
      (a5) a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and
      (b5) a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences; or
   (vi) an antibody comprising
      (a6) a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and
      (b6) a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences.
(Item 18)
   A method of manufacturing an epitope region-bridging biparatopic antibody or an antigen binding fragment or functional equivalent thereof, comprising the steps of:
   (a) providing a population of antibodies to an antigen as an original antibody panel, wherein a number n of antibodies contained in the population of antibodies is a number N of target antibodies to the antigen or greater;
   (b) obtaining binding data for antibodies contained in the original antibody panel;
   (c) clustering the original antibody panel based on the binding data;
   (d) excluding one or more antibodies from the original antibody panel as needed to generate one or more partial panels and clustering each of the one or more partial panels based on the binding data;
   (e) calculating a number e of epitope groups of the original antibody panel and the one or more partial panels, and if there is an original antibody panel or partial panel satisfying e ≥ number E of target epitope groups related to the antigen, selecting the original antibody panel or partial panel satisfying e ≥ E as an epitope normalized antibody panel, but if there is no original antibody panel or partial panel satisfying e ≥ E, adding a new antibody to the original antibody panel or the partial panel to create a new population of antibodies, and repeating (a) to (d);
   (f) selecting two epitopes from a plurality of epitopes included in the obtained epitope normalized antibody panel; and
   (g) linking Fab regions of an antibody that bind to the two epitopes.
(Item 19)
   The method of item 18, wherein step (f) selects the two epitopes so that positions of the two epitopes are on the same side on the antigen.
(Item 20)
   The method of item 18, wherein step (f) selects the two epitopes so that positions of the two epitopes are on opposite sides on the antigen.
(Item A1)
   The epitope region-bridging biparatopic antibody or an antigen binding fragment or functional equivalent thereof of any one of the preceding items, wherein agonistic activity is completely suppressed.
(Item A2)
   The epitope region-bridging biparatopic antibody or an antigen binding fragment or functional equivalent thereof of any one of the preceding items, wherein agonistic activity is enhanced.
(Item A3)
   The epitope region-bridging biparatopic antibody or an antigen binding fragment or functional equivalent thereof of any one of the preceding items for a combination of epitopes required for functioning as an agonist or a combination of epitopes required for functioning as an antagonist.
(Item A4)
   The epitope region-bridging biparatopic antibody or an antigen binding fragment or functional equivalent thereof of any one of the preceding items for preparing an antibody-drug complex (ADC).
(Item A5)
   An antibody-drug complex (ADC) prepared from the epitope region-bridging biparatopic antibody or an antigen binding fragment or functional equivalent thereof of any one of the preceding items.
(Item B1)
   The method of any one of the preceding items for manufacturing an epitope region-bridging biparatopic antibody, or an antigen binding fragment or functional equivalent thereof, with completely suppressed agonistic activity.
(Item B2)
   The method of any one of the preceding items for manufacturing an epitope region-bridging biparatopic antibody, or an antigen binding fragment or functional equivalent thereof, with enhanced agonistic activity.
(Item B3)
   The method of any one of the preceding items for manufacturing an epitope region-bridging biparatopic antibody or an antigen binding fragment or functional equivalent thereof for a combination of epitopes required for functioning as an agonist or a combination of epitopes required for functioning as an antagonist.
(Item B4)
   The method of any one of the preceding items for manufacturing an epitope region-bridging biparatopic antibody or an antigen binding fragment or functional equivalent thereof for preparing an antibody-drug complex (ADC).
(Item B5)
   A method of preparing an antibody-drug complex (ADC) by using the epitope region-bridging biparatopic antibody or an antigen binding fragment or functional equivalent thereof of any one of the preceding items.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

The feature and significant action/effect of the present disclosure other than those described above will be clear to those skilled in the art by referring to the following Detailed Description of the Invention section and the drawings.

### [Brief Description of Drawings]

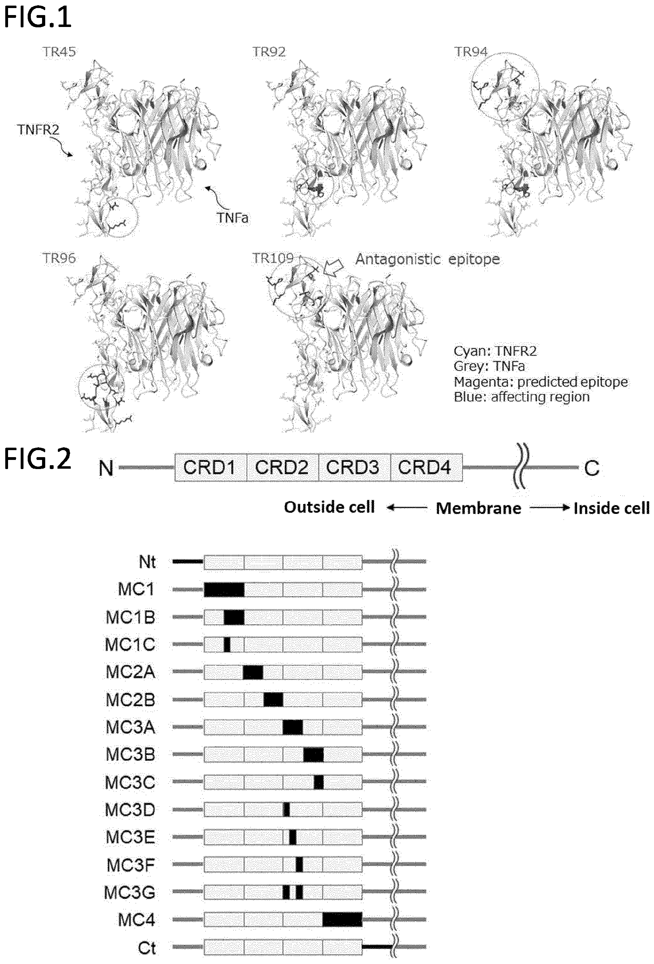

[Figure 1] Figure **1** is an antigen-antibody complex showing the positions of 5 epitopes used for the preparation of an anti-TNFR2 biparatopic antibody in one embodiment of the present disclosure. The substantially spherical shape located on the right side of each complex is the natural ligand TNFα of TNFR2. A vertically long and substantially rod-like shape located on the left side of each complex is TNFR2. Each of the portions encircled by a dashed circle is the epitope.
[Figure 2] Figure **2** is a schematic diagram showing the sites of four cysteine rich domains (CRDs) of TNFR2 and a schematic diagram showing substitution sites of a mutant used in epitope analysis in one embodiment of the present disclosure. TNFR2 has four CRDs, which are known as CRD1, 2, 3, and 4 from the extracellular domain that is distal from the cell membrane. The portion blacked out in the human TNFR2 shown in the lower portion of Figure **2** is replaced with a mouse ortholog sequence. A CRD has a constituent unit known as a module that retains a three-dimensional structure by a disulfide bond. A replacement of an antibody epitope region with a mouse ortholog sequence is designed so that the structure of the module would not be changed significantly.

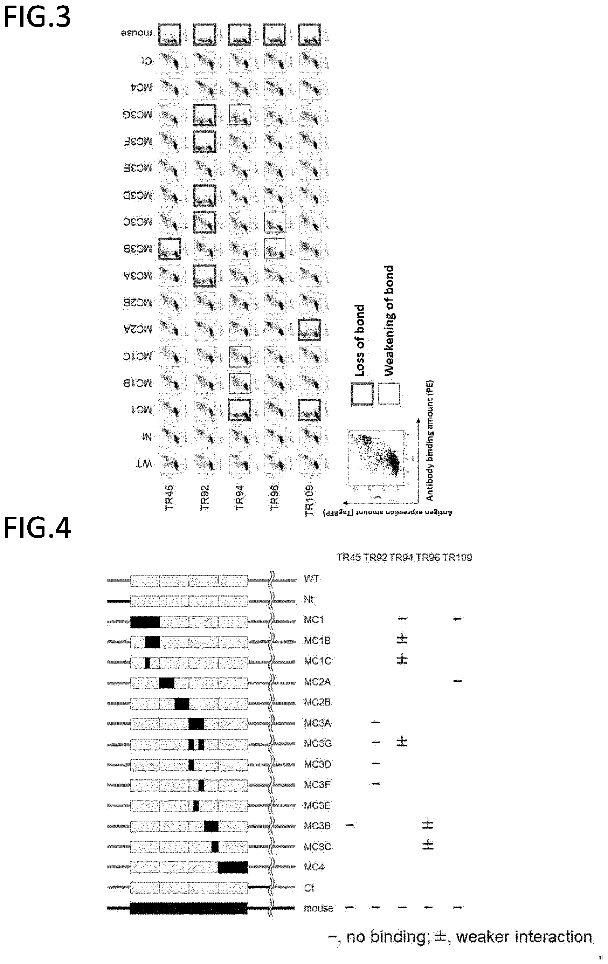

[Figure 3] Figure **3** shows results of flow cytometry (FCM) analysis showing the reactivity of 5 monoclonal antibodies (TR45, TR92, TR94, TR96, and TR109) to each mutant prepared in Figure **2****.** Each TNFR2 mutant was expressed on a 293T cell by transiently expressing an expression plasmid vector encoding each mutant. These expression vectors are designed to express TagBFP via IRES on the same mRNA as a TNFR2 mutant, so that expression levels of an antigen can be monitored as TagBFP fluorescence (vertical axis of the figure). Further, binding of an antibody is detected with a PE-labeled secondary antibody and detected in a fluorescent channel that is different from TagBFP (horizontal axis of the figure). The obtained results can be plotted in 2D as BFP to PE. When an antibody binds specifically, binding level correlates with expression levels of an antigen, and a dot distribution increasing toward the top right side can be observed. Meanwhile, loss of bond was observed in antigen-mutant combinations encircled with a thick frame, and weakening of bond was observed in those encircled with a thin frame. Loss of reactivity of an antibody to these mutants indicates that the epitope structure recognized by each antibody has changed in each mutant. The position of an epitope on a structure was identified from the loss pattern.
[Figure 4] Figure **4** is a schematic diagram showing results of analyzing antigen-antibody affinity from FCM in Figure **3****,** which is associated with sites of substitution of each mutant. In each sequence, blacked-out portions are replaced with a mouse ortholog sequence. Loss of bond with an antibody was observed at portions indicated with a - symbol. A weakening of bond with an antibody was observed at portions indicated with a + symbol.

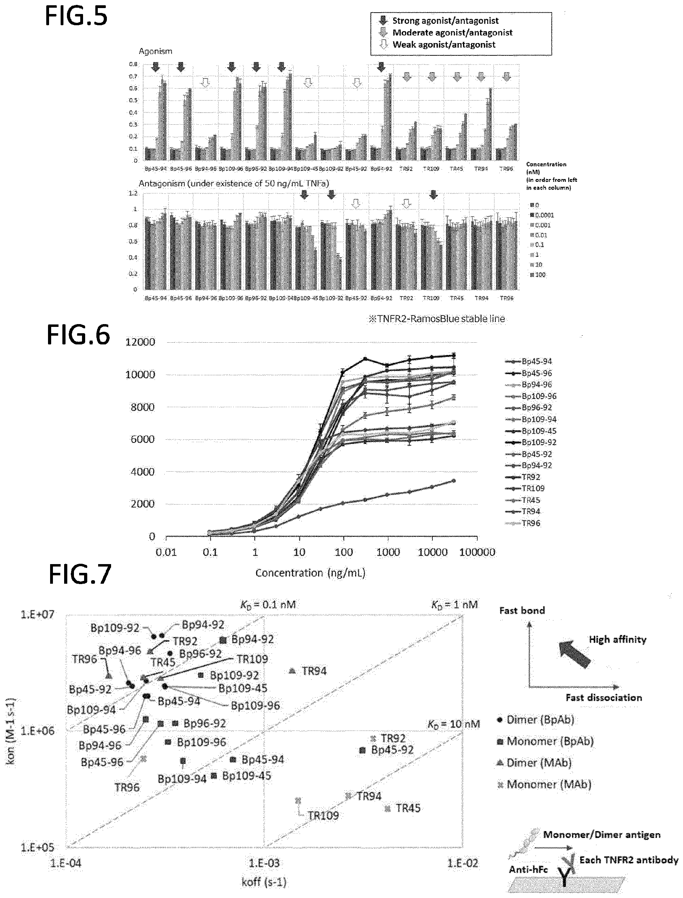

[Figure 5] Figure **5** is a result of a reporter assay in one embodiment of the present disclosure. Results of measuring agonistic activity and antagonistic activity by absorbance using 10 epitope region-bridging biparatopic antibodies and 5 monoclonal antibodies are shown in bar graphs. For agonistic activity, an NFkB-dependent signal induced by stimulation of each antibody by using a Ramos-Blue reporter cell expressing TNFR2 was detected as expression of secreted alkaline phosphatase of a reporter protein by using a PNPP substrate. For antagonistic activity, an NFkB-dependent signal induced by stimulation of a Ramos-Blue reporter cell expressing TNFR2 and TNFR1 with TNFα in the presence of each antibody was detected. The concentrations of each antibody were 0, 0.0001, 0.001, 0.01, 0.1, 1, 10, and 100 nM in each case. The antibodies indicated by a dark arrow are potent agonists or antagonists. The antibodies indicated by a light arrow are moderate agonists or antagonists. The antibodies indicated by a white arrow are weak agonists or antagonists.
[Figure 6] Figure **6** shows results of FCM analysis that evaluated the binding affinity of an epitope region-bridging biparatopic antibody in one embodiment of the present disclosure and monoclonal antibody to a TNFR2 forced expression cell. The concentrations of each antibody were 0.01, 0.1, 1, 10, 100, 1000, 10000, and 100000 ng/mL in each case. The vertical axis indicates the bond of each antibody as Median Fluorescence Intensity after detection thereof with a PE-labeled secondary antibody.
[Figure 7] Figure **7** shows results of evaluating binding affinity of each epitope region-bridging biparatopic antibody in one embodiment of the present disclosure and monoclonal antibody to a TNFR2 monomeric antigen and TNFR2 dimeric antigen by surface plasmon resonance (SPR). A black circle (•) indicates binding affinity between a biparatopic antibody and a dimeric antigen, a black square (■) indicates binding affinity between an epitope region-bridging biparatopic antibody and a monomeric antigen, a black triangle (▲) indicates binding affinity between a monoclonal antibody and a dimeric antigen, and × indicates binding affinity between a monoclonal antibody and a monomeric antigen. In each instance, binding is faster at positions closer to the top, and dissociation is slower at positions closer to the left. Thus, a symbol positioned toward the top left indicates higher affinity.

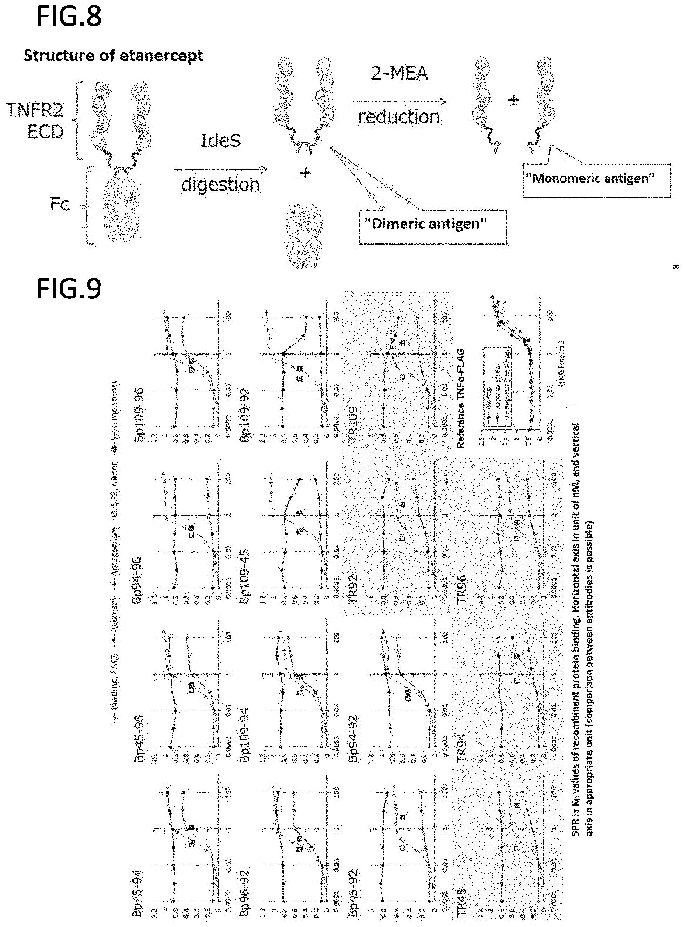

[Figure 8] Figure **8** is a schematic diagram showing the procedure of preparing a TNFR2 monomeric antigen and a TNFR2 dimeric antigen in one embodiment of the present disclosure. A dimer was obtained by digestion of etanercept with an enzyme, and a monomer was obtained by subjecting the dimer to a reductive reoxidation reaction.
[Figure 9] Figure **9** shows graphs that summarize, for each antibody, each of agonistic activity, antagonistic activity, binding activity to TNFR2 forced expression cell according to FCM, and binding activity to a monomeric antigen and dimeric antigen according to SPR of each antibody shown in Figures **5, 6,** and **7**. This allows comparative evaluation between antibodies.

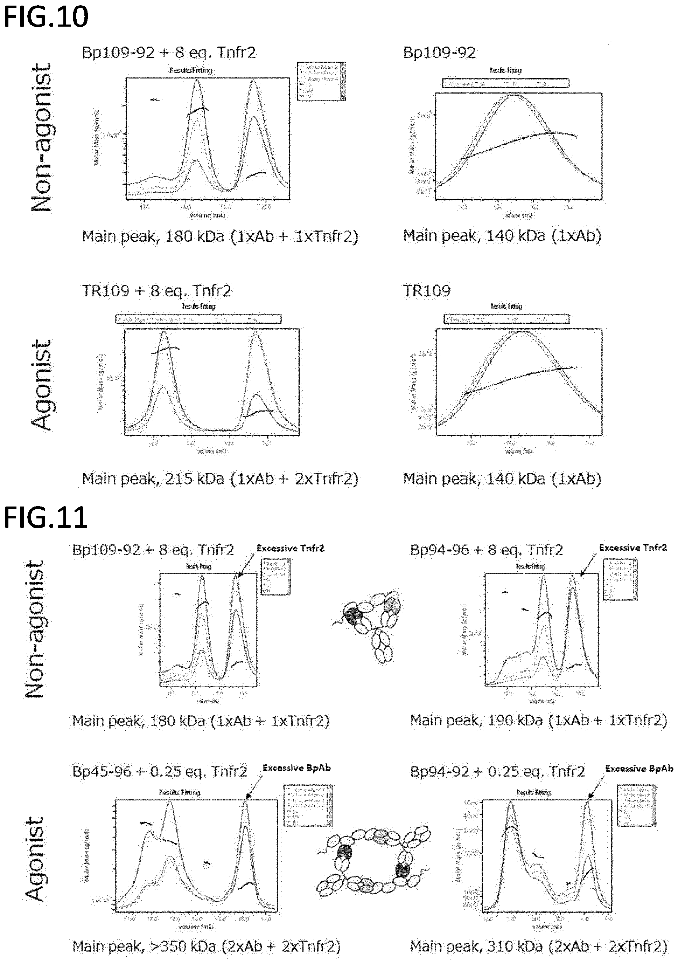

[Figure 10] Figure **10** shows results of connecting size-exclusion chromatography (SEC) and a multi-angle light scattering (MALS) detector to study the states of each antigen-antibody complex. The top row shows results for Bp109-92 (left panel: with antigen, right panel: without antigen), and the bottom row shows results for TR109 (left panel: with antigen, right panel: without antigen). There are two peaks that have eluted in each case with an antigen, showing the molecular weight of an antigen-antibody complex that has eluted first, and only an antigen that has eluted subsequently. Since the molecular weight can be calculated for each elution position, the ratio of the number of molecules of antigen and antibody in an antigen-antibody complex can be estimated. The calculated binding ratio of antibody and antigen is shown below each diagram.
[Figure 11] Figure **11** shows results of SEC-MALS analysis on Bp109-92, Bp94-96, Bp45-96, and Bp94-92. In each case, 8 equivalents or 0.25 equivalents of TNFR2 antigens have been added. The schematic diagrams shown in the middle in each of the top row and bottom row show the form of bond in cases of each of non-agonist and agonist.

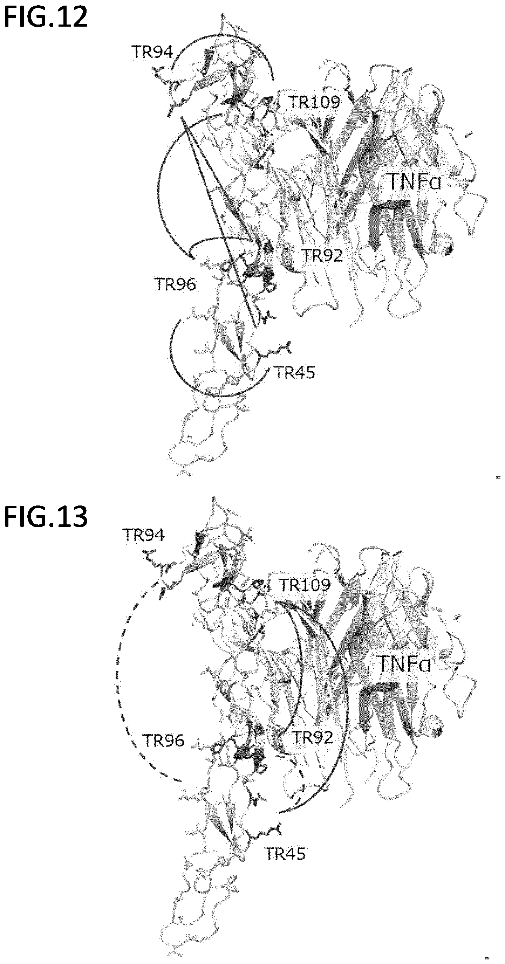

[Figure 12] Figure **12** is a schematic diagram showing a combination of two epitope positions at which six agonist epitope region-bridging biparatopic antibodies (Bp45-96, Bp94-92, Bp109-96, Bp109-94, Bp45-94, and Bp96-92) bind in one embodiment of the present disclosure. The substantially spherical shape located on the right side of the complex is the natural ligand TNFα of TNFR2. A vertically long and substantially rod-like shape located on the left side of the complex is TNFR2. Each of the epitope positions is on opposite sides on the TNFR2 surface in each case.
[Figure 13] Figure **13** is a schematic diagram showing a combination of two epitope positions at which four non-agonist epitope region-bridging biparatopic antibodies (Bp96-94, Bp109-92, Bp109-45, and Bp45-92) bind in one embodiment of the present disclosure. The substantially spherical shape located on the right side of the complex is the natural ligand TNFα of TNFR2. A vertically long and substantially rod-like shape located on the left side of the complex is TNFR2. Each of the epitope positions is on the same side on the TNFR2 surface in each case.

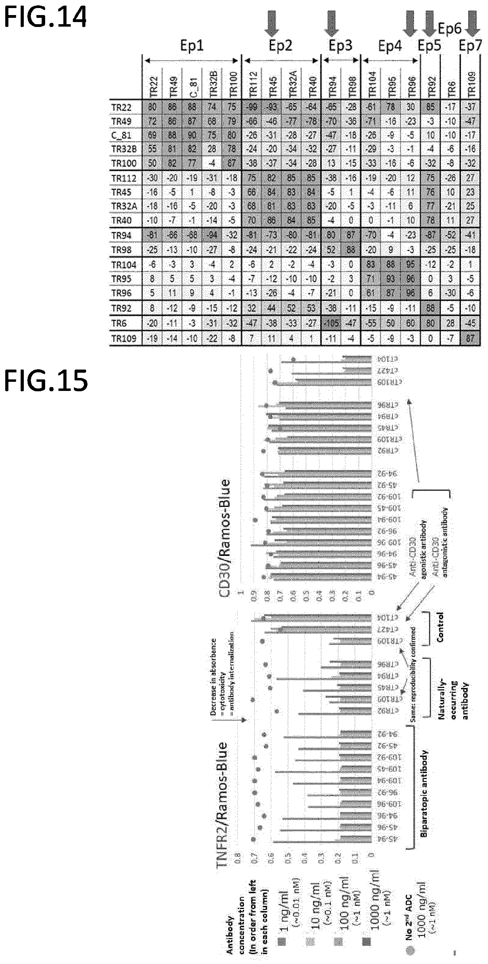

[Figure 14] Figure **14** shows epitopes used in the preparation of an epitope region-bridging biparatopic antibody according to one embodiment of the present disclosure, and an antibody panel showing the epitope group to which the epitopes belong.
[Figure 15] Figure **15** shows a graph comparing the TNFR2-dependent cell internalization capability of an epitope region-bridging biparatopic antibody according to one embodiment of the present disclosure. The concentrations of each antibody were 1, 10, 100, and 1000 ng/mL in each case. A decrease in absorbance indicates cytotoxicity and thus indicates antibody internalization.

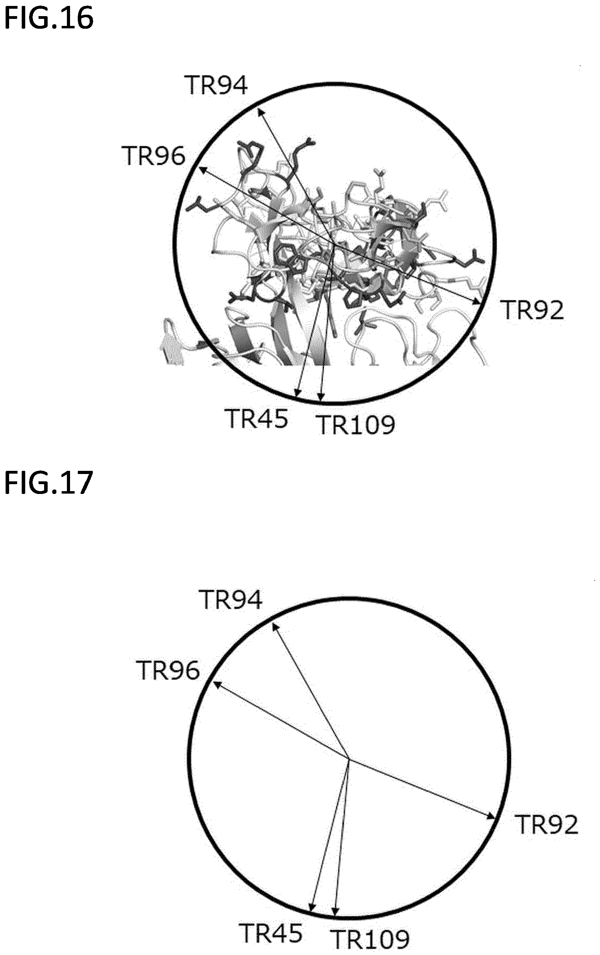

[Figure 16] Figure **16** is a diagram exemplifying a method of preparing a schematic diagram for determining whether two epitope positions on an antigen to which an epitope region-bridging biparatopic antibody according to one embodiment of the present disclosure binds are on the "same side" or "opposite sides".
[Figure 17] Figure **17** is a schematic diagram for determining whether two epitope positions on an antigen to which an epitope region-bridging biparatopic antibody according to one embodiment of the present disclosure binds are on the "same side" or "opposite sides". If, for example, an epitope region-bridging biparatopic antibody is prepared using TR94 and TR96 or TR109 and TR92, the angle formed between vectors in a projection is less than 90°, so that the positions can be determined to be on the "same side". Meanwhile, if, for example, an epitope region-bridging biparatopic antibody is prepared using TR94 and TR109 or TR92 and TR96, the angle formed between vectors in a projection is 90° or greater, so that the positions can be determined to be on "opposite sides".

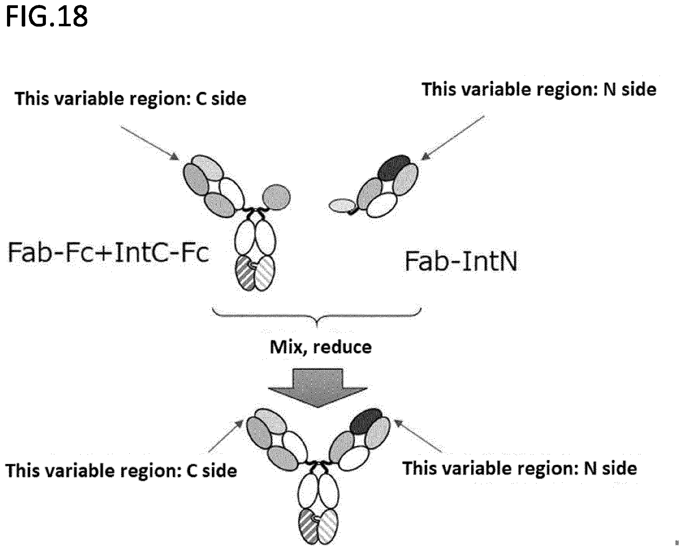

[Figure 18] Figure **18** is a schematic diagram showing the C side and N side upon preparation of an epitope region-bridging biparatopic antibody according to one embodiment of the present disclosure. When a biparatopic antibody is prepared with a Fab region having an Fc region as the C side and a Fab region without an Fc region as the N side for an antibody used as a material, the original Fab region with an Fc region can be referred to as the C side, and Fab region without an Fc region can be referred to as the N side in the prepared biparatopic antibody.

### [Description of Embodiments]

The present disclosure describes the embodiments of Examples of the present disclosure while referring to the drawings.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definitions)

As used herein, "about" refers to a range of ± 10% from the numerical value that is described subsequent to "about".

As used herein, "epitope" refers to a portion on an antigen to which an antibody binds. An epitope is a specific portion of an antigen. If an antigen is a protein of a polypeptide, an epitope can be expressed as a specific amino acid residue in the amino acid sequence of the antigen or a range thereof. An epitope can also be expressed as a population of some of the atoms contained in amino acids constituting the epitope. It is understood that an epitope can be a region smaller than the entire antigen molecule such as an amino acid sequence consisting of several to less than 20 residues.

As used herein, "paratope" refers to a structure derived from Fv of an antibody for specifically binding to one epitope of an antigen. Naturally-occurring IgG antibodies comprise two of the same paratopes (Fv) in one molecule. While divalent as an antibody, the number of paratopes is one (one type).

As used herein, "antigen binding site" refers to a structure comprising one paratope for each antibody molecule to bind to an antigen target. Naturally-occurring IgG antibodies have one paratope (one type), but have two antigen binding sites comprising the same paratope in one molecule, so that such antibodies are divalent.

As used herein, "biparatopic antibody" (BpAb) refers to an artificial antibody, which is derived from two different antibodies to an antigen target, has two types of paratopes exhibiting specific binding affinity to a respective unique epitope, and has one or more antigen binding domains that each independently recognize the two types of paratopes in one molecule for each paratope. A biparatopic antibody can bind to two different epitopes on an antigen.

As used herein, "epitope region" refers to the entire portion on an antigen that cannot be simultaneously bound by an antibody against an epitope present in a certain epitope region and another antibody against a different epitope present in the same epitope region. If the antigen is a protein or a polypeptide, an epitope region can be expressed as a range of positions of amino acids or a population of some atoms contained in such amino acids. Specifically, an antibody against an epitope belonging to a certain epitope region and another antibody to an epitope belonging to the same epitope region do not concurrently bind to an antigen, or may obstruct each other from binding to an antigen. Epitope regions are experimentally determined using a binding assay, but can be estimated by binding simulations. As used herein, "epitope group" refers to a population of one or more epitopes belonging to the same epitope region described above. Antibodies binding to epitopes belonging to the same epitope group exhibit the similar reactivity profile.

As used herein, a biparatopic antibody having two different paratopes derived from two antibodies binding to different epitope regions identified by an epitope normalized antibody panel method is referred to as an epitope region-bridging biparatopic antibody (ERBBA)". An epitope region-bridging biparatopic antibody can form diverse immune complexes by intramolecularly or intermolecularly bridging epitopes. When, for example, an epitope region-bridging biparatopic antibody binds to two different epitopes on a single antigen molecule, an immune complex having an intramolecularly bridged structure is formed. When an epitope region-bridging biparatopic antibody binds to a respective epitope on two antigen molecules, an immune complex having an intermolecularly bridged structure is formed.

As used herein, "agonist" refers to a substance that expresses or enhances a biological action of a receptor for a target entity (e.g., receptor). Examples thereof include naturally-occurring agonists (also known as ligands), synthetic agonists, modified agonists, etc. An antibody having agonistic activity is referred to as an "agonistic antibody".

As used herein, "antagonist" refers to a substance that suppresses or inhibits the expression of a biological action of a receptor for a target entity (e.g., receptor). Examples thereof include naturally-occurring antagonists, synthetic antagonists, modified antagonists, etc. There are antagonists that suppress or inhibit expression, competitively or non-competitively against an agonist (or ligand), etc. An antagonist can also be obtained by modifying an agonist. An antagonist can be encompassed in a concept of a suppressant (inhibitor) or suppressing agent because an antagonist suppresses or inhibits a physiological phenomenon. An antibody having antagonistic activity is referred to as an "antagonistic antibody".

As used herein, "biological action" refers to an action, effect, or function that is inherent to a molecule or activity or change occurring in an organism as a result of the action. Biological action is synonymous with biological effect, organismic effect, and organismic action. For example for antibodies, biological action includes agonistic activity and antagonistic activity described above. For enzymes, biological action includes an effect of suppressing or promoting a chemical reaction catalyzed thereby.

As used herein, "ortholog mapping" refers to a method used in preparing a mutant by extracting a similar structure of an antigen from a molecule having the same ancestral gene as the antigen (i.e., ortholog) in another animal species (e.g., mouse) that is different from an animal where the antigen is derived (e.g., human). An ortholog is a molecule guaranteed to have a conformational structure with a function, as is apparent from exhibiting a biological function similar to an antigen in animals of different species. Thus, when an antigen mutant is prepared by ortholog mapping, the possibility of destruction of the structure of the entire molecule would be much lower compared to common methods of preparing a mutant that do not take orthologs into consideration. As a result, mutants used in ortholog mapping are likely to present the correct conformational structure as the entire molecule, so that the reliability of data can be improved.

As used herein, "fragment" refers to a polypeptide or polynucleotide with a sequence length of 1 to n-1 with respect to a full-length polypeptide or polynucleotide with a length of n. The length of a fragment can be appropriately changed in accordance with the objective. Examples of the lower limit of the length thereof include, in case of polypeptides, 3, 4, 5, 6, 7, 8, 9, 10, 15, 20, 25, 30, 40, 50 and more amino acids. Lengths represented by integers that are not specifically listed herein (e.g., 11, etc.) can also be suitable as a lower limit. Examples of the lower limit of the length thereof include, in case of polynucleotides, 5, 6, 7, 8, 9, 10, 15, 20, 25, 30, 40, 50, 75, 100 and more nucleotides. Lengths represented by integers that are not specifically listed herein (e.g., 11, etc.) can also be suitable as a lower limit. If, for example, a full-length polypeptide or polynucleotide functions as a marker or a target molecule, it is understood that such fragments are within the scope of the present disclosure, as long as the fragments themselves also function as a marker or a target molecule

As used herein, "functional equivalent" with one or more amino acid insertions, substitutions, or deletions, or addition to one or both ends in an amino acid sequence can be used. A modified amino acid sequence can have, for example, 1 to 30, preferably 1 to 20, more preferably 1 to 9, still more preferably 1 to 5, and particularly preferably 1 to 2 amino acid insertions, substitutions, or deletions, or additions to one or both ends. A modified amino acid sequence may preferably be an amino acid sequence having one or more (preferably 1 or several, or 1, 2, 3, or 4) conservative substitutions in the amino acid sequence of TNFR2 or biparatopic antibody of the present disclosure.

Thus, it is understood that a "functional equivalent" of an "epitope region-bridging biparatopic antibody or a fragment thereof" includes, for an antibody, antibodies having TNFR2 binding activity or optionally suppressing activity and fragments thereof themselves, as well as chimeric antibodies, humanized antibodies, multifunctional antibodies, bispecific or oligospecific antibodies, single chain antibodies, scFv, diabodies, sc(Fv)₂, scFv-Fc, etc. Such a "functional equivalent" can bind to the same epitope as the epitope to which a "biparatopic antibody or a fragment thereof" binds.

As used herein, "activity" refers to a function of a molecule in the broadest sense. While not intended to be limiting, activity generally includes biological functions, biochemical functions, physical functions, and chemical functions of a molecule. Examples of activity include enzymatic activity, ability to interact with another molecule, ability to activate, promote, stabilize, inhibit, suppress, or destabilize a function of another molecule, stability, and ability to localize at a specific position within a cell. When applicable, the term also relates to a function of a protein complex in the broadest sense.

As used herein, "decrease", "weakening", or "suppression" of activity, or synonyms thereof refers to a decrease in the quantity, quality, or effect of specific activity, or activity that causes a decrease. Among decreases, "loss" refers to activity, expression product, etc. being less than the detection limit. As used herein, "loss" is encompassed by "decrease" and "suppression".

As used herein, "increase", "enhancement", or "activation" of activity or synonyms thereof refers to an increase in the quantity, quality, or effect of specific activity, or activity that causes an increase. As used herein, the term "activation" also includes states where activity, expression product, etc. less than the detection limit reaching a state where activity, expression product, etc. is at or above the detection limit.

The epitope region-bridging biparatopic antibody or an antigen binding fragment or functional equivalent thereof of the present disclosure can have such a function to suppress or activate a target (e.g., TNFR2, etc.)

### (Description of preferred embodiments)

The preferred embodiments of the present disclosure are described hereinafter. It is understood that the embodiments provided hereinafter are provided to better facilitate the understanding of the present disclosure, and thus the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used alone or in combination.

### Epitope region-bridging biparatopic antibody

The epitope region-bridging biparatopic antibody of the present disclosure is described hereinafter. One embodiment of the present disclosure provides an epitope region-bridging biparatopic antibody or an antigen binding fragment or functional equivalent thereof, having antigen specificity to a first epitope belonging to a first epitope group selected from epitope groups included in each epitope region of a plurality of epitope region groups in an antigen activated by a target molecule binding thereto, and a second epitope belonging to a second epitope group included in an epitope region that is different from the epitope region including the first epitope group, wherein the antibody comprises sequences of a heavy chain variable region and a light chain variable region derived from an antibody to the first epitope, and sequences of a heavy chain variable region and a light chain variable region derived from an antibody to the second epitope.

Thus, in one embodiment of the present disclosure, if the epitope region-bridging biparatopic antibody of the present disclosure has m antigen binding domains to one paratope (wherein m is the maximum value of the number of antigen binding domains to a paratope), it is preferable that a complex having a structure with antigen:antibody of m × n:n (wherein n is the same number that is 1 or greater) is formed by binding to the antigen.

In one embodiment of the present disclosure, if the epitope region-bridging biparatopic antibody of the present disclosure has one antigen binding domain to one paratope, it is preferable that a complex having a structure with antigen:antibody of n:n (wherein n is the same number that is 1 or greater) is formed by binding to the antigen. Since a monoclonal (monoparatopic) antibody is an antibody that targets a specific protein and has specificity to a single antigen epitope, an immune complex having antigen:antibody of 2:1 is formed when bound to an antigen. Meanwhile, a biparatopic antibody can bind to two different epitopes on one or more antigens of a single type. Thus, when binding to two different epitopes on a single antigen molecule to form an immune complex with an intramolecularly bridged structure, antigen:antibody would be 1:1. When binding to respective epitopes on two antigen molecules, antigen:antibody would be n:n (where n is the same number that is 2 or greater). In one embodiment of the present disclosure, antigen:antibody is preferably 1:1, and an immune complex having an intramolecularly bridged structure is formed. When an immune complex having an intermolecularly bridged structure is formed, antigen:antibody is preferably 2:2, 3:3, 4:4, 5:5, 6:6, or 7:7. As long as this can function as a biparatopic antibody of the present disclosure, n is not limited. While n is not limited to an integer, when counting the number individually, the number can be counted as an integer.

In another embodiment of the present disclosure, the epitope region-bridging biparatopic antibody of the present disclosure can have a structure having two or more antigen binding domains to one paratope and the same number of antigen binding domains corresponding to two paratopes, i.e., a structure having two or three each of two different variable regions, such as a structure of IgG-scFv or DVD-Ig. In such a case, the biparatopic antibody of the present disclosure can form a complex having a structure of antigen:antibody of (m × n):n (wherein n is the same number that is 1 or greater) by binding to the antigen, where m is the number of each antigen binding domain. For example, antigen: antibody can be 2:1, 4:2, 6:3, etc. for a tetravalent antibody having two each of two variable regions. If the epitope region-bridging biparatopic antibody of the present disclosure has a different number (i.e., other than two) of antigen binding domains with respect to two paratopes, a complex having a structure with antigen: antibody of (m × n):n can be formed by binding to the antigen in the same manner as the example described above, with such a number which is greater being m (i.e., a maximum value of a number of antigen binding domains to each paratope). For example, antigen: antibody can be 2:1, 4:2, 6:3, etc. for a tetravalent antibody having two each of two variable regions. For hetero-Fc IgG, scFv-scFv, etc., m = 1, so that a complex having a structure with antigen:antibody of n:n (wherein n is the same number that is 1 or greater) described above is formed.

Although not wishing to be bound by any theory, one of the important points in a preferred embodiment of the present disclosure is in obtaining a 1:1 complex with a biparatopic antibody having one each of two variable regions. It can be important that a complex with such a structure exhibits a property that is different from a complex having an antibody bridging two antigen molecules, and preferably can eliminate undesirable activity derived from intermolecular bridging. In one preferred embodiment of the present disclosure, it can be important that agonism is significantly reduced or completely eliminated. In one embodiment using, for example, an antibody as ADC, complete suppression of agonistic activity can be useful. For example, an antibody to a receptor mediating a signal that promotes cancer growth allows cancer cells to grow, but a biparatopic antibody with completely suppressed agonistic activity obtained by the method of the present disclosure is safe when forming an ADC and can be expected to have an antitumor effect. Further, since antibody drugs have a long half-life with a high dosage, the risk thereof cannot be eliminated even with a weak agonistic activity. Meanwhile, a biparatopic antibody obtained by the method of the present disclosure is useful because such an antibody can completely suppress agonistic activity.

In one embodiment of the present disclosure, the molecular weight size and molecular shape of an antigen-antibody complex can be measured by various methods for measuring molecular weight and/or molecular shape that are known in the art. For example, size exclusion chromatography with multi-angle light scattering (SEC-MALS) can analyze particle sizes by static light scattering and obtain information on more absolute molecular weight or molecular shape. Molecular weight may be number average molecular weight (Mn), weight average molecular weight (Mw), or peak molecular weight (Mp). Such molecular weight can also be measured using size exclusion chromatography, other liquid chromatography techniques, static light scattering, or ultrafiltration. In one embodiment of the present disclosure, the molecular shape of an antigen-antibody complex can be captured using an electron microscope.

In one embodiment of the present disclosure, an antigen that is activated by a target molecular binding thereto can be a receptor that initiates a reaction of a cell by binding to a substance (ligand) such as a neurotransmitter, hormone, or cell growth factor, an enzyme that functions as a catalyst for a chemical reaction *in vivo,* etc. In one embodiment of the present disclosure where a receptor is an antigen, although limitation of the types of activity is not intended, the epitope region-bridging biparatopic antibody of the present disclosure has agonistic activity which expresses or enhances a biological action that is inherent to the receptor or has antagonistic activity which suppresses or inhibits expression of a biological action of the receptor by binding to the receptor, which is an antigen. In one embodiment of the present disclosure where an enzyme is an antigen, the epitope region-bridging biparatopic antibody of the present disclosure preferably suppresses or inhibits a chemical reaction that is normally catalyzed by the enzyme. Alternatively, in one embodiment of the present disclosure where an enzyme is an antigen, the epitope region-bridging biparatopic antibody of the present disclosure can promote or enhance a chemical reaction that is normally catalyzed by the enzyme.

In one embodiment of the present disclosure, two epitopes to which the epitope region-bridging biparatopic antibody of the present disclosure binds belong to epitope groups that recognize respectively different epitope regions.

In one embodiment of the present disclosure for the epitope region-bridging biparatopic antibody of the present disclosure that is an antagonist, at least one of the variable regions is derived from an antibody that binds to an epitope group where a binding antibody becomes an antagonist. Meanwhile, the epitope region-bridging biparatopic antibody of the present disclosure that is an agonist is dependent on the relative positional relationship between two epitopes to be bound and is not substantially affected by whether the original antibody that binds to each epitope is an agonist. For example, in one embodiment of the present disclosure where two epitope positions on an antigen to which a biparatopic antibody binds are on opposite sides on the antigen, the biparatopic antibody becomes an agonist. Meanwhile, if the two epitope positions on an antigen to which a biparatopic antibody binds are on the same side on the antigen, the biparatopic antibody would not be an agonist, or agonism can be significantly reduced in a biparatopic antibody having one each of two different paratopes.

In this regard, by configuring two epitope positions on an antigen to which a biparatopic antibody binds to be on the "same side" or "opposite sides", the relative relationship between the two epitope positions on an antigen to which a biparatopic antibody binds and two variable regions of the antibody can be controlled to materialize a desired binding form. For example, selecting two epitope positions on the "same side" on an antigen can enable two variable regions of a single antibody to bind to the two epitopes on a single antigen. Meanwhile, if two epitope positions are on "opposite sides" on an antigen, two variable regions of a single antibody can bind to a total of two epitopes at one location each on two antigens (i.e., one of the epitopes is on antigen A1, while the other epitope is on antigen A2, and antigens A1 and A2 are the same type of antigens). In one embodiment of the present disclosure, how far or how close in distance the two epitope positions are separated can be considered when selecting two epitopes on an antigen to which a biparatopic antibody binds.

For example, in one embodiment of the present disclosure, "same side" or "opposite sides" can be determined by defining a minimum circle comprising all projections when all atoms belonging to four CRD domains are projected onto any plane from a three-dimensional structure of a TNFR2 antigen molecule. In such a case, projection is selected so that the area of the circle is minimized. The direction of an epitope is determined by the following procedure from the projection. First, point B on the circumference closest to each atom is found for all atoms contained in a region replaced with an amino acid sequence derived from an ortholog in each mutant (circle according to the definition described above). Next, point X on the circumference is defined for each antibody to calculate the length of arc BX, where for each antibody, the length of arc BX derived from a mutant found to have loss of reactivity is given a two-fold weighting, and length of arc BX derived from a mutant found to have weakening of reactivity is given a one-fold weighting (i.e., length of arc BX at point B derived from a region with "-" in Figure **4** is ×2, and length of arc BX at point B derived from a region with "±" is ×1). The length of arc BX at point B derived from a region found to have no weakening in reactivity in a mutant is given 0. If an amino acid residue in a naturally-occurring TNFR2 antigen corresponding to a region replaced with an amino acid sequence derived from an ortholog in each mutant is Arg, Tyr, Phe, or Trp, the length of arc BX derived from an atom belonging to the amino acid is given a two-fold weighting, and the length of arc BX derived from an atom belonging to another amino acid is given a 1.5-fold weighting. Point X that minimizes the sum of arc BX at all B is used as center point A of epitope of the specific antibody. Vector OA that passes through center point A of epitope defined for each antibody in this manner and center O of the minimum circle previously defined is obtained for each epitope (Figure **16**). If the scalar product of vectors is a positive value for two epitopes, i.e., the angle formed by vectors in a projection when viewed from the bottom surface of a cylinder while assuming a TNFR2 antigen as a cylinder is less than 90°, two epitopes are determined to be on the "same side". If the scalar product of vectors is 0 or a negative value, epitopes can be determined to be on "opposite sides" (Figure **17**).

In one embodiment of the present disclosure where positions of two epitopes or two epitope regions to which the epitopes belong are close in distance, even if two epitope positions on the "same side" on an antigen are selected, two variable regions of an epitope region-bridging biparatopic antibody compete to inhibit simultaneously binding, so that the epitope region-bridging biparatopic antibody cannot bind to a single antigen. In such a case, the effect of selecting two epitope positions on an antigen to which the epitope region-bridging biparatopic antibody bind to be on the "same side" cannot be obtained. In another embodiment where it is desired to select two epitope positions on the "same side" on an antigen, combinations of antibody variable regions to an epitope where competitive inhibition can result, when arranged on a biparatopic antibody molecule in this manner, can be excluded.

In one embodiment of the present disclosure, the epitope region-bridging biparatopic antibody of the present disclosure can bind, to the antigen, at two epitopes on a side where a normal target molecule of an antigen (e.g., naturally-occurring ligand) binds. Specifically, in such a case, two epitope positions on the antigen to which the epitope region-bridging biparatopic antibody binds would be on the same side on the antigen. Meanwhile, in one embodiment of the present disclosure, the epitope region-bridging biparatopic antibody of the present disclosure can also bind, to the antigen, at a first epitope on the side where a normal target molecule of an antigen binds and a second epitope on the side where a target molecule does not bind. Specifically, in such a case, two epitope positions on an antigen to which an epitope region-bridging biparatopic antibody binds are on the opposite side from a ligand binding site on the antigen.

In one embodiment of the present disclosure, the epitope region-bridging biparatopic antibody of the present disclosure has enhanced agonistic activity compared to the original monoclonal antibody. When two epitope positions to to which the original monoclonal antibody binds are on opposite sides on an antigen from each other in the epitope region bridging biparatopic antibody of the present application, the epitope region-bridging biparatopic antibody would be an agonistic antibody. In such a case, the agonistic activity can be significantly more enhanced than agonistic activity in the original monoclonal antibody.

A naturally-occurring monoclonal antibody retains both agonistic activity and antagonistic activity. For example, TR109 among the monoclonal antibodies to TNFR2 (TR92, TR109, TR45, TR94, and TR96) shown in Figure **5** has a potent antagonistic activity, while also retaining a moderate agonistic activity. In this manner, a naturally-occurring monoclonal antibody may be "an antibody which is an antagonist while also being an agonist" and exhibit a complex behavior depending on the balance of both activities. In one embodiment of the present disclosure, the epitope region-bridging biparatopic antibody of the present disclosure can be configured so that the antibody does not have a property as an agonist or antagonist. For this reason, the epitope region-bridging biparatopic antibody of the present disclosure can be configured as, for example, an antibody with a suppressed agonistic activity and a significantly enhanced antagonistic activity.

In one embodiment of the present disclosure, the epitope region-bridging biparatopic antibody of the present disclosure can enhance or suppress an inherent biological action of an antigen. Thus, if an antigen is, for example, an enzyme, the epitope region-bridging biparatopic antibody of the present disclosure can completely suppress or inhibit a chemical reaction naturally catalyzed by the enzyme.

### Method of preparing an epitope normalized antibody panel

Epitope normalized antibody panels described herein that are used in the present disclosure may be those described in WO 2017/143838 or those with the following modification based on the description therein. For example, the full-length sequences of antigens are clustered in units retaining a higher order structure of an antigen required for an antibody binding function based on sequence analysis on the antigens. Next, a limited number of partial mutant antigen series is prepared to theoretically cover an epitope region by replacing each cluster with a sequence of an antigen homolog, such as an ortholog or paralog. A change in the binding strength of each antibody to each mutant is quantified as a degree of loss of affinity to obtain a parameter from standardizing affinity of each antibody in the same manner as the "epitope normalized antibody panel" technology described in WO 2017/143838. In this regard, this is utilized to obtain information reflecting only epitope positions, not affinity. The resulting reactivity profile is grouped by various resolutions. By using this, resolution can be set in units of functional epitope in the same manner as the "epitope normalized antibody panel" described in WO 2017/143838 to arrive at an "epitope normalized antibody panel" containing positional information.

In this manner, an "epitope normalized antibody panel" used in the present disclosure comprising positional information may be used. It is understood that a manufacturing method of this panel constitutes a part of the present disclosure.

First, the full-length sequences of antigens are clustered in units retaining a higher order structure of an antigen required for an antibody binding function based on sequence analysis on the antigens. Next, a limited number of partial mutant antigen series is prepared to theoretically cover an epitope region by replacing each cluster with a sequence of an antigen homolog, such as an ortholog or paralog. A change in the binding strength of each antibody to each mutant is quantified as a degree of weakening of affinity to obtain a parameter related to epitope position from standardizing affinity of each antibody. The resulting reactivity profile can be grouped by various resolutions to add positional information to an "epitope normalized antibody panel".

In one specific embodiment where ortholog mapping described herein is used in the epitope normalized antibody panel described herein, the degree of loss of affinity becomes nearly 1 or 0 in all antibodies, and can be clearly confirmed, upon use of a mutant of a domain size in a mouse ortholog. Division of an epitope region of a panel antibody matches the pattern of weakening of affinity of each antibody to a mutant. For example, when selecting an antibody from the epitope normalized antibody panel described herein, an antibody is selected as a reference antibody by a baseline such as an antibody having an advantage as a substance, such as high affinity or high stability of an antibody molecule, an antibody that is humanized, or an antibody with an Fv sequence that is determined prior to others, and the antibody can be used as a material for the epitope region-bridging biparatopic antibody of the present disclosure. In such a case, if all positions of epitope regions are suitably dispersed with a certain distance for antibodies in an epitope normalized antibody panel, it can be expected that new interactions can be created when an epitope region-bridging biparatopic antibody is prepared using paratopes thereof, so that this is desirable.

In one embodiment of the present disclosure, it is preferable that a higher order structure of an antigen to be bound is known or can be estimated with a certain degree of precision upon designing the epitope region-bridging biparatopic antibody of the present disclosure. In one embodiment of the present disclosure, it is preferable to obtain, as positional information of an epitope of an antigen, the "direction" (e.g., "same side" or "opposite sides" described herein) or distance information.

In one embodiment of the present disclosure where ortholog mapping is used, the full-length of an ortholog can also be used in a part of a homolog (ortholog and paralog) mapping method. In another embodiment where there is no affinity of an antibody to be measured to an ortholog derived from a mutant and the domain structure of an antigen is clear, an ortholog of an immune host without antigenicity (i.e., mouse ortholog) can be used for a partial exchange. In such a case, all antibodies would not exhibit affinity to a mouse ortholog.

As the epitope normalized antibody panel described herein that can be used in the present disclosure, an antibody panel comprised of a plurality of antibodies that homogenously recognize a large number of epitope regions on a target using sequential binding pattern unique to an antibody group as an indicator can be used. If an antibody group with the recognized epitope regions that are normalized is used, the possibility of functional expression by an antibody of an antigen can be efficiently investigated without omission. In such a case, positional information of an antigen binding site is not obtained, and a paired sequential assay between antibodies is required. Meanwhile, it is understood that this issue is resolved by using homolog mapping. Specifically, an epitope normalized panel can be prepared by newly acquiring data on reactivity of each antibody to an ortholog of an antigen (gene group with homologous functions in a different organism arising from species differentiation from an ancestral gene in common with the antigen) or paralog (gene group with homologous structures that is present as separate molecules in an organism from which the antigen originates and has descended by evolution from an ancestral gene in common with the antigen) in place of a paired sequential assay reaction value between antibodies and using the data as characteristic value of each antibody. In one embodiment of the present disclosure, a conventional epitope normalized antibody panel method can be improved in this manner. An epitope normalized antibody panel method improved in this manner is also within the scope of the present disclosure. Although not wishing to be bound by any theory, such an improvement of an epitope normalized antibody panel method does not require a sequential binding assay, can complete an antibody panel only with antibody reactivity data, and has an advantageous of being capable of estimating the position of an epitope region on an antigen structure.

When developing an antibody, an animal experiment is conducted. Thus, in many cases, a surrogate antibody that exhibits cross-reactivity or binds to a topologically the same epitope region is required, so that ortholog mapping exemplified herein is useful. In one embodiment of the present disclosure, the full-length of a homolog can be used in order to obtain a reaction value for preparing an epitope normalized panel. A normal conformation of an antigen is guaranteed by using an ortholog or paralog, and use of a mutant with mutations at a plurality of locations at the same time can reduce the number of mutants required for determining normalization of epitopes to a practical level. It is understood that a parameter obtained by reactivity to a homolog cannot be directly linked to positional information. For example, when an antigen has epitope regions A-B-C-D and a mouse ortholog has regions A-b-C-d, regions with different reactivity (B ≠ b, D ≠ d) can be detected, but positional information cannot be found only from such detection. For this reason, it is necessary to first define the range of change in the frequency and location of mutations in a homolog in order to obtain an estimated value used in clustering. In this regard, in one embodiment of the present disclosure, positional information of an epitope can be obtained by introducing the concept of affinity loss by taking into consideration the ratio of wide-type antigens to mutant antigens for the affinity of each mutant and antibody.

In one embodiment of the present disclosure, a biparatopic antibody can be prepared by various methods, such as a method described in Scientific Reports volume 7, 8360 (2017) or International Publication No. WO 2017/14383. With such a method, a biparatopic antibody without mispairing of heavy chain/heavy chain and light chain/heavy chain can be prepared by incorporating two parent antibody fragments into a hinge region (four disulfide bonds linking heavy chain/heavy chain and light chain/heavy chain) of an antibody through protein trans-splicing by split inteins. This method can prepare an antibody that can be directly used without a recombinant tag remaining in a product by utilizing an intein, which is a peptide chain recombinant sequence of a bacterial protein DnaE.

Since a biparatopic antibody is prepared by linking Fab regions of two antibodies, an Fc region would be derived from one of the antibodies, and the linker moiety would be asymmetric. In one embodiment of the present disclosure, an Fc region of the biparatopic antibody of the present disclosure may be derived from either antibody. For this reason, either of the two original antibodies can be the N side or C side. Even if the N side and C side are switched, the same property can be exhibited. As for the N side or C side for an antibody used as a material, if a biparatopic antibody is prepared with a Fab region having an Fc region as the C side and a Fab region without an Fc region as the N side as shown in for example Figure **18****,** the original Fab region having an Fc region can be referred to as the C side, and the Fab region without an Fc region can be referred to as the N side in the prepared biparatopic antibody.

In one embodiment of the present disclosure, the positional relationship of two epitopes to which a biparatopic antibody binds can be determined by a method such as ortholog mapping. The method is not particularly limited, as long as epitope positions on an antigen can be identified. The size of an epitope to which an antibody actually binds is a very limited range. Ortholog mapping prepares a mutant based on such a size and classifies the reactivity of an antibody. For this reason, antibodies classified into the same epitope group exhibit the same reactivity profile. On the other hand, alanine scanning, etc. performs mapping using a mutant with a location of mutation that is too small compared to the recognition range of an antibody. Thus, even for antibodies that are in the same epitope group, individual antibodies would each exhibit different reactivity, reaching an obvious conclusion of having different physical epitopes for all antibodies. Conversely, if a large mutant such as a deletion mutant exceeding the size of an epitope is used for mapping, a number of epitope groups would exhibit the same reactivity, so that epitopes that should be normally classified in separate epitope groups are included in the same epitope group. Specifically, in one embodiment of the present disclosure, ortholog mapping data can be used as data providing accurate positional information for an epitope group by suitably controlling the size of a mutant.

All normal antibodies are produced within the body of an animal. Since an immune host exhibits immunotolerance to autoantigens, the repertoire of resulting antibodies would be biased to a portion that does not react to an autoantigen. In one embodiment of the present disclosure, ortholog mapping can create a mutant series by using a similar structure of an epitope where such self-tolerance is occurring. Specifically, since loss of reactivity of an antibody is used as an indicator, the most efficient mutant panel is prepared, so that positional information of a functional epitope group can be obtained dramatically more efficiently than other methods.

In one embodiment of the present disclosure, the epitope region-bridging biparatopic antibody of the present disclosure can be for a membrane protein as an antigen, especially for a membrane protein belonging to a TNF receptor super family. Tumor necrosis factor (TNF) receptor super family (TNFRSF) is one of the membrane glycoprotein groups that act to regulate various cellular functions. TNFRSF has one or more cysteine rich domains (CRDs) in the extracellular region, and such CRDs are structurally similar to one another. Furthermore, each CRD is configured to be characterized by a structural unit based on a disulfide bond between cysteines known as a module structure, and these receptors share about 20 to 30% of homology of the whole. Many TNFRSFs are type I membrane proteins (having the N-terminus outside of the cell, and C-terminus within the cell).

In one embodiment of the present disclosure, the epitope region-bridging biparatopic antibody of the present disclosure is for TNFR2 as an antigen. TNFR2 is one type of a TNFR super family (SF) and is a receptor having TNFα as a ligand (Croft M1, Benedict CA, Ware CF. Clinical targeting of the TNF and TNFR superfamilies. Nat Rev Drug Discov. 12(2), 147-68. (2013)).; Grell M, Douni E, Wajant H, Lohden M, Clauss M, Maxeiner B, et al. The transmembrane form of tumor necrosis factor is the prime activating ligand of the 80 kDa tumornecrosis factor receptor. Cell. 83(5), 793-802. (1995).). TNFα binds to two receptors TNFR1 and TNFR2 and is known to serve a critical role in various physiological functions in the body (following #1 to #18).

#1 Lubrano di Ricco M, Ronin E, Collares D, Divoux J, Gregoire S, Wajant H, Gomes T, Grinberg-Bleyer Y, Baud V, Marodon G, Salomon BL, Tumor necrosis factor receptor family co-stimulation increases regulatory T cell activation and function via NF-kappaB, Eur J Immunol 2020
#2 Chou CK, Chen X, Preferential Expansion of CD4(+)Foxp3(+) Regulatory T Cells (Tregs) In Vitro by Tumor Necrosis Factor, Methods Mol Biol 2111 (71-78, 2020
#3 Atretkhany KN, Gogoleva VS, Drutskaya MS, Nedospasov SA, Distinct modes of TNF signaling through its two receptors in health and disease, J Leukoc Biol 2020
#4 Wajant H, Beilhack A, Targeting Regulatory T Cells by Addressing Tumor Necrosis Factor and Its Receptors in Allogeneic Hematopoietic Cell Transplantation and Cancer, Front Immunol10 (2040, 2019
#5 Torrey H, Khodadoust M, Tran L, Baum D, Defusco A, Kim YH, Faustman DL, Targeted killing of TNFR2-expressing tumor cells and Tregs by TNFR2 antagonistic antibodies in advanced Sezary syndrome, Leukemia 33(5): 1206-1218, 2019
#6 Tam EM, Fulton RB, Sampson JF, Muda M, Camblin A, Richards J, Koshkaryev A, Tang J, Kurella V, Jiao Y, Xu L, Zhang K, Kohli N, Luus L, Hutto E, Kumar S, Lulo J, Paragas V, Wong C, Suchy J, Grabow S, Dugast AS, Zhang H, Depis F, Feau S, Jakubowski A, Qiao W, Craig G, Razlog M, Qiu J, Zhou Y, Marks JD, Croft M, Drummond DC, Raue A, Antibody-mediated targeting of TNFR2 activates CD8(+) T cells in mice and promotes antitumor immunity, Sci Transl Med 11(512):2019
#7 Medler J, Wajant H, Tumor necrosis factor receptor-2 (TNFR2): an overview of an emerging drug target, Expert Opin Ther Targets 23(4): 295-307, 2019
#8 Chen X, Plebanski M, Editorial: The Roleof TNF-TNFR2 Signal in Immunosuppressive Cells and Its Therapeutic Implications, Front Immunol 10 (2126, 2019
#9 Al-Hatamleh MAI, E ARE, Boer JC, FerjiK, Six JL, Chen X, Elkord E, Plebanski M, Mohamud R, Synergistic Effects of Nanomedicine Targeting TNFR2 and DNA Demethylation Inhibitor-An Opportunity for Cancer Treatment, Cells 9(1): 2019
#10 Zou H, Li R, Hu H, Hu Y, Chen X, Modulation of Regulatory T Cell Activity by TNF Receptor Type II-Targeting Pharmacological Agents, Front Immunol 9 (594, 2018 Sheng Y, Li F, Qin Z, TNF Receptor 2 Makes Tumor Necrosis Factor a Friend of Tumors, Front Immunol 9 (1170, 2018
#11 Shaikh F, He J, Bhadra P, Chen X, Siu SWI, TNF Receptor Type II as an Emerging Drug Target for the Treatment of Cancer, Autoimmune Diseases, and Graft-Versus-Host Disease: Current Perspectives and In Silico Search for Small Molecule Binders, Front Immunol9 (1382, 2018
#12 Salomon BL, Leclerc M, Tosello J, Ronin E, Piaggio E, Cohen JL, Tumor Necrosis Factor alpha and Regulatory T Cells in Oncoimmunology, Front Immunol 9 (444, 2018
#13 Nie Y, He J, Shirota H, Trivett AL, Yang, Klinman DM, Oppenheim JJ, Chen X, Blockade of TNFR2 signaling enhances the immunotherapeutic effect of CpG ODN in a mouse model of colon cancer, SciSignal 11(511): 2018
#14 Fischer R, Proske M, Duffey M, Stangl H, Martinez GF, Peters N, Kraske A, Straub RH, Bethea JR, Kontermann RE, Pfizenmaier K, Selective Activation of Tumor Necrosis Factor Receptor II Induces Antiinflammatory Responses and Alleviates Experimental Arthritis, Arthritis Rheumatol 70(5): 722-735, 2018
#15 Faustman DL, TNF, TNF inducers, and TNFR2 agonists: A new path to type 1 diabetes treatment, Diabetes Metab Res Rev34(1): 2018
#16 Torrey H, Butterworth J, Mera T, Okubo Y, Wang L, Baum D, Defusco A, Plager S, Warden S, Huang D, Vanamee E, Foster R, Faustman DL, Targeting TNFR2 with antagonistic antibodies inhibits proliferation of ovarian cancer cells and tumor-associated Tregs, Sci Signal 10(462): 2017
#17 Williams GS, Mistry B, Guillard S, Ulrichsen JC, Sandercock AM, Wang J, Gonzalez-Munoz A, Parmentier J, Black C, Soden J, Freeth J, Jovanovic J, Leyland R, Al-Lamki RS, Leishman AJ, Rust SJ, Stewart R, Jermutus L, Bradley JR, Bedian V, Valge-Archer V, Minter R, Wilkinson RW, Phenotypic screening reveals TNFR2 as a promising target for cancer immunotherapy, Oncotarget 7(42): 68278-68291, 2016
#18 Okubo Y, Torrey H, Butterworth J, Zheng H, Faustman DL, Treg activation defect in type 1diabetes: correction with TNFR2 agonism, Clin Transl Immunology 5(1):e56, 2016

Meanwhile, the details of the function of TNFR2 have not been elucidated. Diligent studies for elucidating what function it has and the role thereof are ongoing. Expression of TNFR2 is limited to vascular endothelial cells, T cell populations comprising lymphocytes, etc. (Ware, C.F. et al. Tumor necrosis factor (TNF) receptor expression in T lymphocytes. Differential regulation of the type I TNF receptor during activation of resting and effector T cells. J. Immunol. 147, 4229-4238 (1991).), and TNFR2 activation dependent T cell growth and improved cell viability have been reported (Kim EY, Priatel JJ, Teh SJ, Teh HS. TNF receptor type 2 (p75) functions as a costimulator for antigen driven T cell responses in vivo. Journal of immunology. 176(2), 1026-35. (2006).). In view of the above, TNFR2 is expected to have a major role in controlling inflammation and in the biological defense mechanism, but elucidation of the detailed function is expected to lead to the use of TNFR2 as a target of a therapeutic drug for development and exacerbation of pathological conditions (#7).

TNFR2 is a membrane receptor that is highly expressed in regulatory T cells (hereinafter Treg) contributing to immunosuppression. It is known that a signal mediated by TNFR2 is important in the growth and function of Treg. TNFR2, when activated by binding of an endogenous ligand (TNFα), is known to contribute to the growth of Treg and expression of immunosuppression through a transcription factor NFκB. Such biological activities can also be typically included.

As used herein, "tumor necrosis factor receptor 2 (TNFR2)" is a type of receptor, which is also denoted as TNFRII, TNFRSF1B, CD120b, TBPII, TNF-R-II, TNF-R75, TNFBR, TNFR1B, TNFR80, p75, p75TNFR, tumor necrosis factor receptor superfamily member 1B, etc. Tumor necrosis factor (TNF)α binds thereto to mediate signaling. As for the genetic information thereof, RefSeq (mRNA) is NM_001066 (human) and NM_011610 (mouse). For the gene product thereof, in view of the accession numbers described in NCBI, RefSeq (Protein) is NP_001057.1 (human), NP_035740 (mouse), or NP_035740.2 (mouse). Physiological action of TNF is expressed via a TNF receptor (TNFR) that is widely present in cells in the body except in red blood cells. TNFR includes TNFR1 (p60) and TNFR2 (p80). It is reported that affinity to TNFR2 is 5-fold higher than affinity to TNFR1. Much like TNF, TNFR is present while forming a trimer. TNFR1 is constitutively expressed in many tissues in the entire body, while TNFR2 is an induced receptor that is expressed in a cell of an immune system via some type of stimulation. TNFR is involved in infection defense and antitumor action by elevating antibody production, while TNFR is involved in autoimmune diseases such as rheumatoid arthritis and psoriasis, etc.

As described above, it is known that a signal mediated by TNFR2 is important in the growth and function of regulatory T cells (hereinafter Treg) contributing to immunosuppression. Since TNFR2 is known to contribute to Treg growth and expression of immunosuppression through transcription factor NFκB when activated by binding of an endogenous ligand (TNFα), administration of an anti-TNFR2 antagonist antibody is promising as a novel cancer therapy method by impairing Treg infiltrating into cancer tissue to promote antitumor immunity of a patient.

Besides Treg, TNFR2 is also specifically highly expressed in different types of immunocompetent cells such as myeloid-derived suppressor cells (MDSCs) and mediates important growth signals. It is understood that an anti-TNFR2 antibody suppresses the growth of these cells, resulting in disabling immunosuppression and promoting cancer immunity to be useful in cancer therapy.

Furthermore, TNFR2 is expressed at a high level in some cases in many types of cancer cells in multiple myeloma, colon cancer, ovarian cancer, etc. in many cases. Since it is well known that cancer cell growth is promoted through activation of NFkB downstream of TNFR2, it is understood that an antitumor effect is exhibited if these cancer cells are impaired with an anti-TNFR2 antibody.

To impair Treg, MDSC, cancer cells, etc. expressing TNFR2, an antibody which inhibits binding of an endogenous ligand by antibody binding and exhibits a function as an antagonist that does not produce an intracellular signal is useful. Cytotoxic actions such as ADCC (antibody-dependent cell-mediated cytotoxicity) and ADCP (antibody-dependent cell-mediated cytotoxicity and complement dependent cytotoxicity) induced by an antibody binding to antigen and then to an Fc receptor of an immune cell such as an NK cell are understood as a likely effector function of the antibody. Complement dependent cytotoxic actions induced by an antibody bound to an antigen binding to a complete via an Fc region are also expected.

Thus, anti-TNFR2 antagonistic antibodies are expected to be applied to therapeutic drugs, which promotes cancer immunity from not only competing against an endogenous ligand TNFα to inhibit the growth or function of Treg, MDSC, TNFR2 positive cancer cells, etc., but also damaging these cells by the effector action of the antibodies to impair TNFR2 expressing regulatory T cells or MDSCs, as well as to antibody drugs, which impairs TNFR2 positive cancer cells themselves.

A therapy that enhances the growth or function of Treg to suppress immunity is promising for diseases due to excessive immunity such as autoimmune diseases. An antibody which exhibits an agonistic function that induces an intracellular signal similar to an endogenous ligand due to antibody binding is useful for enhancing the growth or function of Treg.

For example, agonist antibodies are expected to have a therapeutic effect, particularly on intractable autoimmune diseases among TNF related diseases. Examples thereof include diseases such as Crohn's disease, Sjogren's syndrome, multiple sclerosis, type I diabetes, lupus erythematosus (SLE), ankylosing spondylitis, and chronic rheumatoid arthritis. In addition, it is expected that agonist antibodies can be utilized for a disease expected to have a protective action on a certain neurological disease (e.g., repairing hippocampus, protecting retinal neuron, etc.). TNFR2 agonists are demonstrated to be possibly useful in the improvement of a pathological condition by correcting dysfunction of Treg and suppressing autoimmunity in pathological conditions of type I diabetes (Ref #18).

It may be possible to grow Treg by utilizing an anti-TNFR2 agonist and harvest Treg in the body out of the body and culture the Treg, and use the amplified immunosuppressive Treg cells in autologous cell transfusion therapy or adoptive immunotherapy after returning the Treg into the body of a patient (Ref #2).

In one embodiment of the present disclosure, the original monoclonal antibody of an epitope region-bridging biparatopic antibody to TNFR2 is not particularly limited, as long as the monoclonal antibody can bind to one of the epitope regions with TNFR2 as an antigen. Examples thereof include TR92, TR109, TR45, TR94, and TR96 shown in Figure **14****.** The antibodies denoted with TR or C in Figure **14** are all monoclonal antibodies to TNFR2. When preparing, for example, an epitope region-bridging biparatopic antibody to TNFR2, any combination of the monoclonal antibodies belonging to different epitope regions may be used.

For example, in one embodiment of the present disclosure, an epitope region-bridging biparatopic antibody to TNFR2 as an antigen can be prepared using two monoclonal antibodies that recognize different epitope regions for TNFR2, and such a combination may be any combination. A biparatopic antibody has two antibodies from which it is derived and is thus asymmetric as described above. Meanwhile, either paratope derived from the original two antibodies may be the N side or C side. For example for epitope region-bridging biparatopic antibodies to TNFR2, an epitope region-bridging biparatopic antibody obtained by using TR96 as a sequence on the N side and TR94 as a sequence on the C side (Bp96-94), and an epitope region-bridging biparatopic antibody prepared by switching the N side and C side (Bp94-96) exhibit the same property.

In one embodiment of the present disclosure, an epitope region-bridging biparatopic antibody to TNFR2 can comprise a heavy chain variable region and light chain variable region of two antibodies selected from for example TR92, TR109, TR45, TR94, and TR96. In another embodiment, an epitope region-bridging biparatopic antibody to TNFR2 can comprise heavy chain CDRs (CDR1, CDR2, and CDR3) and light chain CDRs (CDR1, CDR2, and CDR3) of two antibodies selected from for example TR92, TR109, TR45, TR94, and TR96.

In one embodiment of the present disclosure, two monoclonal antibodies from which an epitope region-bridging biparatopic antibody is derived may be of any combination as described above, but the property of a prepared epitope region bridging biparatopic antibody changes in accordance with the two selected monoclonal antibodies. For example, in one embodiment of the present disclosure where an epitope region-bridging biparatopic antibody is prepared from TR92 and TR109, antagonistic activity is highly enhanced, while agonistic activity is significantly weakened or removed. This is associated with the relative positions of epitopes to which each monoclonal antibody binds. If an epitope region-bridging biparatopic antibody is prepared from TR45 and TR94, TR45 and TR96, TR109 and TR96, TR96 and TR92, TR109 and TR94, or TR94 and TR92, agonistic activity is enhanced. This is also associated with the relative positions of epitopes to which each monoclonal antibody binds.

The heavy chain and light chain and respective CDR1, CDR2, and CDR3 of each antibody have the following sequences (according to the definition of Kabat).

**[Table 1]**

| | | Protein Sequence | CDR1 | CDR2 | CDR3 |
|---|---|---|---|---|---|
| TR109 | VH | | VYGVN | | DGRRYALDY |
| TR109 | VL | | | RASNLDS | QQSNEDPYT |
| TR45 | VH | | GYNMN | | |
| TR45 | VL | | | YTSILHS | QQGNTLPWT |
| TR92 | VH | | ESIIH | | HEGPYVYFDY |
| TR92 | VL | | | YWTSTRHT | QHHYSTPYT |
| TR94 | VH | | SYWMQ | | DGt4YYAMDY |
| TR94 | VL | | | STSNLAS | HQYHRSPPT |
| TR96 | VH | | DTYMH | | TQLYY |
| TR96 | VL | | | STSNLAS | HQYHRSPLT |

In one embodiment of the present disclosure, the sequences of heavy chain and light chain and their respective CDR1, CDR2, and CDR3 of each monoclonal antibody can each have an amino acid sequences with sequence identity of at least about 50%, about 60%, about 70%, about 80%, preferably at least about 90%, more preferably at least about 95%, about 96%, about 97%, about 98%, or about 99%, and most preferably 100% to the sequences described in Table 1.

"CDR" is a complementarity-determining region amino acid sequence of an antigen binding protein, which is a hypervariable region of immunoglobulin heavy chain and light chain. A variable moiety of an immunoglobulin has three heavy chain CDRs and three light chain CDRs (or CDR regions). Accordingly, the "CDR" herein refers to three heavy chain CDRs, three light chain CDRs, or all heavy chain and light chain CDRs.

In one embodiment of the present disclosure, amino acid residues of an antibody sequence such as a heavy chain, light chain, or variable domain sequence can be numbered in accordance with the Kabat numbering scheme. In another embodiment, a numbering scheme other than the Kabat numbering scheme can be used. For example, a numbering scheme that is well known in the art such as Chothia, Aho, or IMGT can be used. Accordingly, in one embodiment of the present disclosure, amino acid residues of an antibody sequence can use a sequence in accordance with any numbering scheme or use the minimum overlapping region in accordance with two or more numbering schemes as a minimum binding unit.

In one embodiment of the present disclosure, the epitope region-bridging biparatopic antibody of the present disclosure can utilize high affinity to an antigen and long half-life to be internalized within a cell as an antibody-drug complex (ADC). ADC formation with the epitope region-bridging biparatopic antibody of the present disclosure can internalize an antibody within a cell and release a low molecular weight compound in a form with activity in a metabolic system such as a lysosome to kill cells. Thus, the epitope region-bridging biparatopic antibody of the present disclosure can target an intracellular protein.

The antibody of the present disclosure can be provided as an antibody having two or more functions. Such a biparatopic antibody having two or more functions are also referred to as a multifunctional biparatopic antibody. In one embodiment of the present disclosure, the epitope region-bridging biparatopic antibody of the present disclosure can be prepared while maintaining the internalization capability of the original monoclonal antibody. For this reason, the epitope region-bridging biparatopic antibody of the present disclosure can, for example, have significantly enhanced antagonistic activity as well as an internalization capability. Thus, even when internalization such as ADC formation is required, the epitope region-bridging biparatopic antibody of the present disclosure is useful.

In one specific embodiment, the antibody of the present disclosure can be provided as an antibody having two or more functions. Such a biparatopic antibody having two or more functions is also referred to as a multifunctional biparatopic antibody.

In one embodiment of the present disclosure, the epitope region-bridging biparatopic antibody of the present disclosure can be prepared as an antibody having a biological action of interest by selecting two epitope positions to which it binds (e.g., epitope positions on the "same side" or "opposite sides", "intramolecular bridging" or "intermolecular bridging" of an antigen-antibody complex, etc.) so that the biological action of interest can be exerted, such as an antibody with enhanced or suppressed agonistic activity or an antibody with enhanced or suppressed antagonistic activity. Such a biological action of interest of the epitope region-bridging biparatopic antibody of the present disclosure does not affect internalization through ADC formation. Thus, in one embodiment of the present disclosure, the epitope region-bridging biparatopic antibody of the present disclosure can be an antibody having a biological action requiring internalization of a target protein.

### (General technology)

The molecular biological methodology, biochemical methodology, microbiological methodology, and bioinformatics used herein are well known in the art. Any well-known or conventional methodology can be used.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the each document is specifically described in its entirety.

As described above, the present disclosure has been described while showing preferred embodiments to facilitate understanding. The present disclosure is described hereinafter based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### (Examples)

The present disclosure is described in more detail hereinafter while using the Examples, but the present disclosure is not limited to such Examples.

The experimental methods and materials used in the present disclosure are described hereinafter. While the embodiments use the following experimental methods, the same results can also be obtained by using other experimental methods. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, etc.)

### (Example 1: Ortholog mapping for the preparation of an anti-TNFR2 epitope region-bridging biparatopic antibody)

An epitope normalized antibody panel to TNFR2 was prepared by the method disclosed in Example 4 of WO 2018/092907 (Figure **14**). In this Example, a reference antibody representing each of the seven obtained epitope regions was selected, and the sequences of reference monoclonal antibodies targeting five epitopes were identified. An anti-TNFR2 epitope region-bridging biparatopic antibody was prepared from a human IgG1 constant region sequence by using the identified Fv sequence. The five reference monoclonal antibodies include TR45 belonging to Ep2, TR94 belonging to Ep3, TR96 belonging to Ep4, TR92 belonging to Ep5, and TR109 belonging to Ep7 (Figure **14**). Figure **1** shows five epitopes used in the preparation of an anti-TNFR2 biparatopic antibody. Each of the portions encircled with a dashed circle is an epitope.

As disclosed in the schematic diagram of Figure **2****,** TNFR2 has four cysteine rich domains (CRD), which are known as CRD1, 2, 3, and 4 from the extracellular domain that is distal from the cell membrane (Croft M, Benedict CA, Ware CF. Clinical targeting of the TNF and TNFR superfamilies. Nat Rev Drug Discov. 12(2), 147-68. (2013).; Mukai Y, Nakamura T, Yoshikawa M, Yoshioka Y, Tsunoda S, Nakagawa S, Yamagata Y, Tsutsumi Y. Solution of the structure of the TNF-TNFR2 complex. Sci Signal.3 (148), ra83. (2010).) (hereinafter, domains 1, 2, 3, and 4). TNFR2 ligand TNFα is activated by binding to domain 2 and domain 3 (Grell M, Douni E, Wajant H, Lohden M, Clauss M, Maxeiner B, Georgopoulos S, Lesslauer W, Kollias G, Pfizenmaier K, Scheurich P. The transmembrane form of tumor necrosis factor is the prime activating ligand of the 80 kDa tumor necrosis factor receptor. Cell. 83(5), 793-802. (1995).). It is known that a classical pathway where activation induces degradation of IKB retaining NFκB (p50/RelA) in the cytoplasm to induce intranuclear migration of NFκB (p50/RelA), and a non-classical pathway where p100 of NFκB (p100/RelB) is phosphorylated to induced processing, resulting in NFκB (p52/RelB) and intranuclear migration are activated (Naude PJ, den Boer JA, Luiten PG, Eisel UL. Tumor necrosis factor receptor cross-talk. FEBS J.278(6), 888-98. (2011).). Meanwhile, an anti-TNFR2 antibody series uses the full-length of an extracellular moiety of TNFR2 in screening for separating specific antibodies and immunity, and thus could bind to sites other than a ligand binding site. The epitope recognized by the antibody and the function of the antibody were unknown.

Ortholog mapping was performed to study the positional relationship of epitopes targeted by the five monoclonal antibodies used on TNFR2. The DNA sequence with the blacked-out portion of human TNFR2 replaced with a mouse ortholog sequence in Figure **2** was prepared. The DNA sequence that has transfected a cell is bound to an antibody, and the reactivity with a naturally-occurring monoclonal antibody was compared. The replaced portion was deemed as an epitope site of the antibody by using weakened reactivity of a mutant through ortholog mapping compared to a naturally-occurring monoclonal antibody as an indicator.

For preparation of a mutant, a DNA sequence was prepared by replacing largely a CRD domain, as one unit, with a mouse ortholog sequence. A DNA sequence was prepared by replacing, more finely, a region comprised of one or more turns, or a sheet region flanked by two turns with a mouse ortholog sequence. 10 or less amino acids were replaced with a KOD-Plus-Mutagenesis Kit (Toyobo), and replacement with a longer chain was performed by amplifying a mouse sequence DNA and human sequence DNA through PCR using KOD-Plus-Neo (Toyobo) and incorporating the DNA with NEBuilderHiFi DNA Assembly Master Mix (New England Biolabs).

### (Example 2: Reactivity test using a mutant)

HEK 293T cells were passaged using a Dulbecco's Modified Eagle Medium (hereinafter, DMEM) comprising 100 FBS and 1% PS under the conditions of 37°C and 5% CO₂. The HEK 293T cells were adjusted to 5 × 10⁵ cells/ml, added to a 6-well flat bottom plate (Costar) at 2 ml/well, and cultured overnight under the conditions of 37°C and 5% CO₂. HEK 293T was transfected with TNFR2-IRES-TagBFP in accordance with the methodology of PEI MAX (Polysciences, Inc.) and cultured overnight under the conditions of 37°C and 5% CO₂. HEK 293T treated with 0.53 mM trypsin comprising 0.05% EDTA was adjusted to 8 × 10⁵ cells/ml using a FACS buffer (0.1% sodium azide, 2.5% FBS, PBS solution) and added to a 96-well V bottom plate (Violamo) at 25 pl/well. After separating transiently expressing cells, each TNFR2 antibody adjusted to 1.5 µg/ml was added at 25 pl/well and left standing for 1 hour on ice to allow interaction. After centrifugation at 1200 rpm for 5 minutes, the supernatant was removed. After washing once with a FACS buffer, anti-mouse IgG-PE diluted 200-fold was added at 25 pl/well, and the sample was left standing for 30 minutes away from light on ice. After centrifugation at 1200 rpm for 5 minutes, the supernatant was removed. After washing once with a FACS buffer, the sample was suspended at 200 pl/well and analyzed by flow cytometer (FCM).

Figure **3** shows the result of FCM analysis. Figure **4** shows this data correlated with positional data for ortholog mapping. As shown in Figures **3** and **4****,** a TR45 monoclonal antibody has lost reactivity with an antigen in an MC3B mutant having a part of a CRD3 region replaced. It was found in view of the above that an epitope site on TNFR2 to which the TR45 monoclonal antibody binds is in the CRD3 region. Likewise, the epitope sites on TNFR2 to which each of the monoclonal antibodies TR92, TR94, TR96, and TR109 binds were identified. Each epitope site corresponds to the portion encircled with a dashed circle in Figure **1****.**

### (Example 3: Preparation of a biparatopic antibody)

A biparatopic antibody was prepared by a modified methodology of a method described in Han, L., Chen, J., et al. Efficient generation of bispecific IgG antibodies by split intein mediated protein trans-splicing system. Sci. Rep., 7, 8360 (2017) and WO 2017/143838 by using five monoclonal antibodies (TR45, TR94, TR96, TR92, and TR109) that bind to each epitope site identified in the above manner. Specifically, the documents described above prepare a biparatopic antibody without mispairing of heavy chain/heavy chain and light chain/heavy chain by incorporating two parent antibody fragments into a hinge region of an antibody through a protein trans-splicing by split inteins. This method prepares an antibody that can be directly used without a recombinant tag remaining in a product by utilizing an intein, which is a peptide chain recombinant sequence of a bacterial protein DnaE.

First, an "N side" fragment was prepared in the following manner. Expi293F cells (Thermo Fisher Scientific) were introduced with a plasmid encoding an "N side" light chain and a plasmid incorporated with a DNA sequence introduced with 6× His tag, maltose-binding protein (MBP), and Cfa DnaE IntN (Stevens, A.J., Brown, Z. Z., Shah, N.H., Sekar, G., Cowburn, D., Muir, T. W., J. Am. Chem. Soc. 138, 2162-2165 (2016)) downstream of an "N side" Fab region heavy chain (VH-CH1), and the cells were subjected to shake culture for 6 to 7 days under conditions of 37°C, and 8% CO₂. An N side fragment was recovered from the medium supernatant with Complete His-Tag Purification Resin (Roche Diagnostics) and purified by size exclusion chromatography with HiLoad Superdex 200 26/600 pg (GE Healthcare).

The "C side" was prepared in the following manner. A mutant reported in Merchant, A. M., Zhu, Z., Yuan, J. Q., Goddard, A., Adams, C. W., Presta, L. G. and Carter, P. An efficient route to human bispecific IgG. Nat. Biotechnol., 16, 677-681 (1998) was utilized for heterodimerization of Fc. In the same manner as reported in Akiba, H., Satoh, R., Nagata, S., Tsumoto, K., Antib. Ther., 2, 65-69 (2019), a "C side" Fab region heavy chain (VH-CH1), hinge, and mutant Fc-comprising gene was prepared for a knob chain. A 6× His tag, MBP, Cfa DnaE IntC, hinge and mutant Fc-comprising gene was prepared for a hole chain. To the two plasmids, a plasmid encoding a "C side" light chain was added and introduced into Expi293F cells (Thermo Fisher Scientific), which were subjected to shake culture for 6 to 7 days under conditions of 37°C and 8% CO₂. A C side fragment was recovered from the medium supernatant with Complete His-Tag Purification Resin (Roche Diagnostics) and purified by size exclusion chromatography with HiLoad Superdex 200 26/600 (GE Healthcare).

A biparatopic antibody was mixed with 15 µM of N side fragment, 10 µM of C side fragment, and 2 mM of dithiothreitol, and incubated for 2 hours at 37°C. The mixture passed through Amylose resin (New England Biolabs) to remove unreacted components and byproducts was purified by size exclusion chromatography with Superdex 200 Increase 10/300 (GE Healthcare).

Combinations of monoclonal antibodies in a biparatopic antibody prepared in the manner described above are shown in the following Table 2.

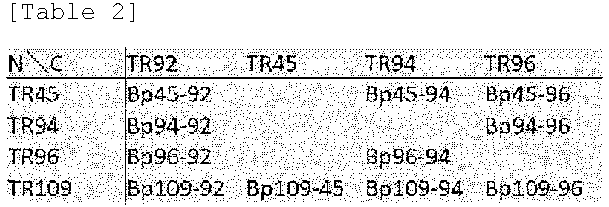

Since a biparatopic antibody (antibody indicated by Bp) links Fab regions of two antibodies, an Fc region would be derived from one of the antibodies. For this reason, strictly speaking, the linker moiety would be asymmetric, but there was no difference in activity as expected, even if the N side and C side were switched in Bp94-96 and Bp96-94 prepared using TR94 and TR96. In the following Examples, Bp94-96 was used.

### (Example 4: Reporter assay)

Agonistic activity and antagonistic activity were measured using 10 epitope region-bridging biparatopic antibodies prepared in the above manner and original monoclonal antibodies as comparative examples.

First, agonistic activity was measured in the following manner. An NFkB dependent signal downstream of TNFR2 induced by adding each antibody by using a Ramos-Blue reporter cell expressing TNFR2 was detected as expression of secreted alkaline phosphatase of a reporter protein by a color method using a p-nitrophenylphosphate (pNPP) substrate. For both the biparatopic antibodies and monoclonal antibodies, recombinant human chimeric antibodies obtained by using the Expi293 cell line were used.

2 × 10⁵ cells were suspended to be 100 pl in IMDM comprising 10% FBS and added to each well of a 96-well culture plate. Each antibody was added to each well at concentrations of 0, 0.0001, 0.001, 0.01, 0.1, 1, 10, and 100 nM and cultured overnight. On the next day, hydrolysis of pNPP used as a coloring substance by enzymatic activity of alkaline phosphatase secreted in culture supernatant of cells in an NFkB dependent manner by stimulation of each antibody was measured through changes in absorbance. The mean of three measurements of absorbance was calculated. Measurement results obtained by adding each antibody were evaluated from "absorbance without antibody or TNFα" for agonist activity or from "absorbance without antibody with TNFα" for antagonistic activity. The above experiment was independently conducted three times. The mean and standard deviation are displayed as bar graphs. The top row of Figure **5** shows the results of measuring agonistic activity.

Next, the functional antagonistic activity of each antibody was investigated by stimulating Ramos-Blue reporter cells expressing TNFR2 with TNFR2 endogenous ligand TNFα (50 ng/mL) and studying the suppression of NFkB dependent signals of each antibody in the presence of TNFα in the same experiment system.

Ramos-Blue reporter cells express endogenous TNFR1 in addition to TNFR2 introduced for expression. Thus, both TNFR2 and TNFR1 are activated when stimulated with an endogenous ligand TNFα. Therefore, the functional antagonistic effect by an anti-TNFR2 antibody is a partial effect mediated only by TNFR2. The bottom row of Figure 5 shows the results of measuring antagonistic activity.

For agonistic activity, all monoclonal antibodies used exhibited moderate agonism. Meanwhile, it was found that epitope region-bridging biparatopic antibodies are separated into those exhibiting a potent agonistic activity and those exhibiting a weak agonistic activity. Specifically, a total of six biparatopic antibodies, i.e., Bp45-94, Bp45-96, Bp109-96, Bp96-92, Bp109-94, and Bp94-92, exhibit a very potent agonistic activity, which was 10-fold or more potent agonistic activity compared to TR94 exhibiting the most potent agonistic activity among monoclonal antibodies. Specifically, it can be understood that these six epitope region-bridging biparatopic antibodies are effective as an agonistic antibody even at low concentrations.

Meanwhile, antagonistic activity of an epitope region-bridging biparatopic antibody was dependent on antagonistic activity in the original monoclonal antibody. Specifically, a prepared biparatopic antibody did not become an antagonistic antibody unless at least one of the two original monoclonal antibodies of the biparatopic antibody was an antagonistic antibody. Since agonistic activity is completely suppressed in Bp109-92 among epitope region-bridging biparatopic antibodies that are antagonistic antibodies, it can be understood to be more effective than the original TR109 as an antagonistic antibody.

### (Example 5: Evaluation of bond to forced expression cell)

Subsequently, binding affinity of each antibody was evaluated using Ramos-Blue reporter cells expressing TNFR2. Each antibody was added at concentrations of 0.01, 0.1, 1, 10, 100, 1000, 10000, and 100000 ng/mL to the TNFR2-Ramos-Blue cells used in a reporter assay, and the cells were incubated for 1 hour on ice. After centrifugation for 5 minutes at 1200 rpm, the supernatant was removed. After washing once with a FACS buffer, anti-human IgG-PE was added to each well. The samples were left standing for 30 minutes away from light on ice. After centrifugation for 5 minutes at 1200 rpm, the supernatant was removed. After washing once with a FACS buffer, fluorescence was analyzed with a flow cytometer (FCM). The mean value of fluorescence intensity was used as a value corresponding to the amount of binding. Experiments were independently conducted twice under the same condition.

For 10 antibodies other than those with a variable region of TR94 (Bp45-94, Bp94-96, Bp109-94, Bp94-92, and TR94), k that minimizes Σᵢ(kAi^{1st}-Ai^{2nd})² was found, wherein Ai is the mean value of fluorescence intensity of three on the high concentration side (plateau region) (i = 1, 2, ... 10; corresponding to each antibody). This value was used to level the two experimental data. Figure **6** shows the mean value and standard deviation for each plot. It was found that affinity to an antigen was nearly the same for each antibody, except for TR94 (line at the bottom).

### (Example 6: Evaluation of reactivity to a recombinant antigen)

Binding kinetics analysis and evaluation of each antibody to a monomeric antigen and dimeric antigen were conducted through surface plasmon resonance (SPR) using BIAcore T200 system (GE Healthcare). Monomeric and dimeric antigens were prepared. The affinity of each antibody to the monomeric and dimeric antigens was evaluated to study the presence/absence of intramolecular bridging activity. The SPR technology is based on measuring the index of refraction near the surface of a gold coated biosensor chip. A change in the index of refraction indicates a change in mass on the surface due to an interaction between an immobilized antibody and a monomeric or dimeric antigen infused into the solution. The mass increases when a molecule binds to an antibody immobilized on the surface, whereas the mass decreases when an analyte dissociates from an immobilized antibody. Analysis of a biparatopic antibody and the original monoclonal antibody by SPR can not only evaluate the performance of each antibody, but also see the effect of multivalent bond. Specifically, multivalent bond is important for those that change significantly from ■ to ● shown in Figure 7 for an epitope region-bridging biparatopic antibody, and those that change significantly from × to ▲ shown in Figure **7** for a monoclonal antibody.

The monomeric and dimeric antigens were prepared as shown in the schematic diagram of Figure **8****.** First, an etanercept (Pfizer), which is an Fc fusion protein of a TNFR2 extracellular region, was prepared, and a TNFR2 antigen molecule was obtained by enzymatic degradation and reductive re-oxidation reaction. An antibody was immobilized on a sensor chip using a human antibody capture kit according to the protocol by using a BIAcore T200 system (GE Healthcare). The interaction was analyzed by using the two types of antigens described above as analytes. Table 3 shows the SPR parameters of each antibody, and Figure **7** shows summarized results in a graph.

**[Table 3]**

| Atas | Ag | ka (1/Ms) | kd (1/s) | KD(M) |
|---|---|---|---|---|
| TR45 | Dimer | 2.93E+06 | 2.45E-04 | 3.39E-11 |
| TR94 | Dimer | 3.31E+06 | 0.001354 | 4.18E-10 |
| TR96 | Dimer | 2.99E+06 | 1.64E-04 | 5.49E-11 |
| TR92 | Dimer | 4.88E+06 | 2.66E-04 | 5.44E-11 |
| TR109 | Dimer | 2.85E+06 | 3.00E-04 | 1.05E-10 |
| Bp45-94 | Dimer | 2.00E+06 | 2.59E-04 | 1.29E-10 |
| Bp45-96 | Dimer | 2.01E+06 | 2.51E-04 | 1.25E-10 |
| Bp94-96 | Dimer | 2.58E+06 | 2.07E-04 | 8.01E-11 |
| Bp109-45 | Dimer | 2.41E+06 | 3.16E-04 | 1.31E-10 |
| Bp109-94 | Dimer | 2.71E+06 | 2.55E-04 | 9.41E-11 |
| Bp109-96 | Dimer | 2.45E+06 | 3.15E-04 | 1.28E-10 |
| Bp96-92 | Dimer | 4.67E+O6 | 3.36E-04 | 7.19E-11 |
| Bp45-92 | Dimer | 2.42E+06 | 2.16E-04 | 3.91E-11. |
| Bp94-92 | Dimer | 6.62E+06 | 3.05E-04 | 4.60E-11 |
| Bpl09-92 | Dimer | 6.48E+06 | 2.78E-04 | 4.29E-11 |
| TR45 | Monomer | 2.20E+05 | 0.004125 | 1.88E-08 |
| TR94 | Monomer | 2.77E+05 | 0.002617 | 9.45E-09 |
| TR96 | Monomer | 5.80E+05 | 2.43E-04 | 4.16E-10 |
| TR92 | Monomer | 8.69E+05 | 0.003463 | 3.99E-09 |
| TR109 | Monomer | 2.66E+05 | 0.00148 | 5.56E-09 |
| Bp45-94 | Monomer | 5.77E+05 | 6.92E-04 | 1.20E-09 |
| Bp45-96 | Monomer | 1.17E+06 | 2.99E-04 | 2.55E-10 |
| Bp94-96 | Monomer | 1.28E+06 | 2.53E-04 | 1.98E-10 |
| Bp109-45 | Monomer | 4.19E+05 | 5.54E-04 | 1.32E-09 |
| Bp109-94 | Monomer | 5.63E+05 | 3.88E-04 | 6.89E-10 |
| Bp109-96 | Monomer | 8.06E+05 | 3.28E-04 | 4.07E-10 |
| Bp96-92 | Monomer | 1.18E+06 | 3.54E-04 | 3.01E-10 |
| Bp45-92 | Monomer | 6.96E+05 | 0.003122 | 4.49E-09 |
| Bp94-92 | Monomer | 6.24E+06 | 6.14E-04 | 9.84E-11 |
| Bp109-92 | Monomer | 3.05E+06 | 4.79E-04 | 1.57E-10 |

It can be understood from Figure **7** that monoclonal antibody TR94 has weak binding activity to a dimeric antigen, and TR96 has high binding activity to a monomeric antigen. This indicates an exceptional binding activity among monoclonal antibodies. Further, many biparatopic antibodies had sufficiently potent binding activity to a dimeric antigen, and Bp94-92, Bp109-92, and Bp94-96 in particular exhibited a particularly potent binding activity. Furthermore, just like many monoclonal antibodies, Bp45-92 had weak binding activity to a monomeric antigen, while other biparatopic antibodies exhibited higher affinity than a monoclonal antibody from which it is derived. It was found that this result does not contradict the results of evaluation by FCM described above. An epitope region-bridging biparatopic antibody had comparable or improved antigen specific binding affinity compared to a naturally-occurring antibody.

### (Example 7: Concentration dependent concentration)

Subsequently, the agonistic activity and antagonistic activity of each antibody obtained by the reporter assay in Example 4, the binding activity to TNFR2 expressing cells from flow cytometry in Example 5, and the results of SPR in Example 6 were plotted on the same graph for each antibody to study how binding activity to an antigen and agonistic activity and antagonistic activity change in a concentration dependent manner. Figure **9** shows the results thereof.

### (Example 8: Evaluation of bridging capability by SEC-MALS)

To evaluate what ratio of prepared biparatopic antibodies bind to an antigen, structural analysis was conducted using size exclusion chromatography with multi-angle light scattering (SEC-MALS), which is a more absolute molecular weight measuring method (SEC: Superose 6 10/300, GE Healthcare, MALS: Dawn 8, Wyatt Technology). The molecular weight size of a complex can be found by mixing, and subjecting to SEC-MALS, a recombinant antibody/antigen protein. Analysis was performed in accordance with a common method. Each panel in Figure **10** shows results of SEC-MALS on Bp109-92 and TR109. The top two panels are results for Bp109-92, and the bottom two panels are results for TR109. In the left side panel of each row, 8 equivalents of TNFR2-ECD are mixed with respect to each antibody.

As shown in the top and bottom panels on the left side of Figure **10****,** it can be understood that a SEC-MALS profile contains a peak that elutes first and a peak that elutes later. The lines crossing each peak is representative, and the elution time represents the molecular weight of detected molecular species. It can be understood from the results shown in the Bp109-92 (without agonistic activity) panel on the top left that a 180 kDA peak molecular weight (first peak) was detected when mixed with an antigen TNFR2, and antibodies and antigens were bound at 1:1. It was also found from the bottom left panel that a 215 kDa peak molecular (first peak) was detected when mixed with an antigen TNFR2, and antibodies and antigens were bound at 1:2 as expected for TR109 (with agonistic activity). Specifically, it is understood therefrom that TR109 is a divalent naturally-occurring monoclonal antibody.

Likewise, the binding form of other biparatopic antibodies (Bp94-96, Bp45-96, and Bp94-92) was analyzed using SEC-MALS. Figure **11** shows the results thereof. For Bp94-96 (without agonistic activity) shown in the top right panel of Figure **11****,** it was found that a 190 kDa peak molecular weight (first peak) was detected when mixed with 8 equivalents of TNFR2, and antibodies and antigens were bound at 1:1. For Bp45-96 (with agonistic activity) and Bp94-92 (with agonistic activity) shown in the bottom panel of Figure **11****,** it can be understood that 350 kDa or greater or 310 kDa peak molecular weight (first peak) was detected when each was mixed with 0.25 equivalents of TNFR2, and antibodies and antigens were bound at 2:2.

Table 4 summarizes the results of SEC-MALS on each antibody. Table 5 shows results summarizing the rough molecular weight of each antibody and antigen-antibody complex.

**[Table 4]**

| Ab : TNFR2 | 1:1 | 1:2 | 2 : 2 or more | Other |
|---|---|---|---|---|
| | TR94 | TR109 | Bp45-96 | Bp109-94 |
| | Bp109-92 | TR45 | Bp94-92 | |
| | Bp94-96 | TR92 | Bp109-96 | |
| | Bp109-45 | TR96 | Bp45-94 | |
| | | Bp45-92 | Bp96-92 | |

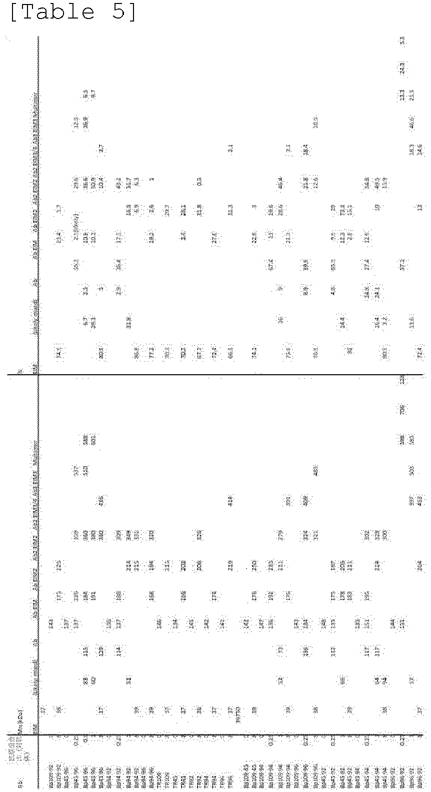

TR94, Bp109-92, Bp94-96, and Bp109-45 form a 1:1 complex. Bp109-92 and Bp109-45 in particular have no agonistic activity, and Bp94-96 exhibited a weak agonistic activity. TR94 exhibits a special bond with no saturation of binding on a cell under the measured concentration conditions (Figure **6**), so that the complex is considered incomplete. TR109, TR45, TR92, TR96, and Bp45-92 form a 1:2 complex. TR109, TR45, TR92, and TR96 in particular are monoclonal antibodies, which exhibited a moderate agonistic activity, and Bp45-92 exhibited a weak agonistic activity. In Bp45-92, epitopes to which TR45 and TR92 bind are adjacent, and placement of antigen binding sites thereof in a biparatopic antibody results in competitive inhibition, so that the antibody may not be bound to a single antigen. Bp45-96, Bp94-92, Bp109-96, Bp45-94, and Bp96-92 that form a 2:2 complex and Bp109-94 exhibiting a concentration dependent behavior exhibited a potent agonistic activity.

It can be understood from these results that an antigen and an antibody binding to form a multimer becomes a potent agonist. Figure **12** shows results of studying a combination of two epitope positions to which 6 epitope region-bridging biparatopic antibodies (forming a complex with antibody-antigen of 2:2 or greater) that are agonists bind. It can be understood that each epitope position is on opposite sides on the TNFR2 surface in each case. Figure **13** shows results of studying a combination of two epitope positions to which four biparatopic antibodies (forming a complex with antibody:antibody of 1:1 or 1:2) that are not agonists bind. The dotted line in Figure **13** is the combination of epitope positions exhibiting a weak agonistic activity. It can be understood that each epitope position is on the same side on the TNFR2 surface in each case.

It can be understood from these results that if epitopes are on "opposite sides" of a TNFR2 antigen, i.e., the opposite side of Fab when viewed from an antigen (base) is arranged not to move away when Fab is bound, this would necessarily be a complex bridging two or more TNFR2 molecules. It was found that in such a case, this would necessarily be an agonist. It was also found that this would not be an agonist if epitopes are on the "same side" of a TNFR2 antigen.

### (Example 9: Comparison of TNFR2 dependent cell internalization of various anti-TNFR2 biparatopic antibodies and naturally-occurring anti-TNFR2 antibodies)

Cell internalization capabilities of prepared anti-TNFR2 biparatopic antibodies and naturally-occurring anti-TNFR2 monoclonal antibodies were compared. The prepared biparatopic antibodies and their parent chimeric antibodies were cultured in the presence of TNFR2/Ramos-Blue cells or CD30/Ramos-Blue cells and secondary ADC. The number of viable cells was counted by the WST-8 method after four days to measure cytotoxicity due to internalization of an antibody. A decrease in absorbance indicates the presence of cytotoxicity and thus antibody internalization. Figure **15** shows the results thereof.

All prepared biparatopic antibodies similarly exhibited a TNFR2 antigen dependent internalization capability. The internalization capability was at the same level as a naturally-occurring antibody, and unrelated to a potent agonist activity or potent antagonistic activity of each biparatopic antibody. It is understood in view of the above that a difference between intracellular bridging and intercellular bridging defining agonism and antagonism does not affect internalization. It is understood that antibody modification requiring internalization such as ADC formation is also effective even for antagonists.

### (Example 10: Potent antitumor activity of epitope region bridging anti-TNFR2 biparatopic antagonistic antibody in an immune responsive tumor model mouse)

TNFR2 humanized mice with a TNFR2 gene on a mouse genome replaced with a human TNFR2 gene are used to compare and evaluate antitumor activity of a naturally-occurring anti-TNFR2 antibody TR109 and an epitope region-bridging biparatopic antibody TR92-TR109 with a potent antagonist activity. In TNFR2 humanized mice, replaced human TNFR2 is localized in regulatory T cells, in the same manner as the expression pattern of a mouse TNFR2 gene in a wild-type mouse, and human TNFR2 functions in place of mouse TNFR2. Specifically, in TNFR2 humanized mice, human TNFR2 receives stimulation from endogenous TNF and regulates the growth and function of regulatory T cells. When mouse cancer cells (e.g., colon cancer derived MC38 cells) are transplanted subcutaneously into TNFR2 humanized mice, the transplanted cells engraft over time, so that tumor grows. When the transplanted tumor has grown to about 100 mm3, mice are separated into four groups. One group is untreated, one group is intraperitoneally administered with negative control human IgG1, one group is intraperitoneally administered with a naturally-occurring antibody TR109, and one group is intraperitoneally administered with an epitope region-bridging biparatopic antibody TR92-TR109. When an increase in tumor after administration is observed over time, a sustained increase in tumor is observed in the untreated group and negative control human IgG1 administration group. Meanwhile, weak tumor growth suppression is detected in the naturally-occurring antibody TR109 administration group, and suspension of tumor growth is observed in the epitope region-bridging biparatopic antibody TR92-TR109 administration group. When regulatory T cells infiltrating tumor tissue of each mouse are checked by tissue staining, regulatory T cells hardly infiltrate tumor tissue in the epitope region-bridging biparatopic antibody TR92-TR109 administration group. Thus, epitope region-bridging biparatopic antibodies achieve an antagonistic effect at a strong level which cannot be achieved with a naturally-occurring antibody, suppress regulatory T cells, and exhibit a potent antitumor effect.

In addition, the present disclosure can be modified in various manners and is not limited to an embodiment described above. The present disclosure can be modified in various manners within the scope that does not change the gist of the invention.

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2020-61014 filed on March 30, 2020 with the Japan Patent Office. It is understood that the entire content thereof is incorporated herein by reference in the same manner as if the contents constitute the content of the present application in its entirety

### [Sequence Listing Free Text]

| | | | | | | | |
|---|---|---|---|---|---|---|---|
| SEQ | ID | NO: | 1: | | TR45 | heavy | chain |
| SEQ | ID | NO: | 2: | | TR92 | heavy | chain |
| SEQ | ID | NO: | 3: | | TR94 | heavy | chain |
| SEQ | ID | NO: | 4: | | TR96 | heavy | chain |
| SEQ | ID | NO: | 5: | | TR109 | heavy | chain |
| SEQ | ID | NO: | 6: | | TR45 | light | chain |
| SEQ | ID | NO: | 7: | | TR92 | light | chain |
| SEQ | ID | NO: | 8: | | TR94 | light | chain |
| SEQ | ID | NO: | 9: | | TR96 | light | chain |
| SEQ | ID | NO: | 10: | | TR109 | light | chain |
| SEQ | ID | NO: | 11: | TR45 | heavy | chain | CDR1 |
| SEQ | ID | NO: | 12: | TR45 | heavy | chain | CDR2 |
| SEQ | ID | NO: | 13: | TR45 | heavy | chain | CDR3 |
| SEQ | ID | NO: | 14: | TR92 | heavy | chain | CDR1 |
| SEQ | ID | NO: | 15: | TR92 | heavy | chain | CDR2 |
| SEQ | ID | NO: | 16: | TR92 | heavy | chain | CDR3 |
| SEQ | ID | NO: | 17: | TR94 | heavy | chain | CDR1 |
| SEQ | ID | NO: | 18: | TR94 | heavy | chain | CDR2 |
| SEQ | ID | NO: | 19: | TR94 | heavy | chain | CDR3 |
| SEQ | ID | NO: | 20: | TR96 | heavy | chain | CDR1 |
| SEQ | ID | NO: | 21: | TR96 | heavy | chain | CDR2 |
| SEQ | ID | NO: | 22: | TR96 | heavy | chain | CDR3 |
| SEQ | ID | NO: | 23: | TR109 | heavy | chain | CDR1 |
| SEQ | ID | NO: | 24: | TR109 | heavy | chain | CDR2 |
| SEQ | ID | NO: | 25: | TR109 | heavy | chain | CDR3 |
| SEQ | ID | NO: | 26: | TR45 | light | chain | CDR1 |
| SEQ | ID | NO: | 27: | TR45 | light | chain | CDR2 |
| SEQ | ID | NO: | 28: | TR45 | light | chain | CDR3 |
| SEQ | ID | NO: | 29: | TR92 | light | chain | CDR1 |
| SEQ | ID | NO: | 30: | TR92 | light | chain | CDR2 |
| SEQ | ID | NO: | 31: | TR92 | light | chain | CDR3 |
| SEQ | ID | NO: | 32: | TR94 | light | chain | CDR1 |
| SEQ | ID | NO: | 33: | TR94 | light | chain | CDR2 |
| SEQ | ID | NO: | 34: | TR94 | light | chain | CDR3 |
| SEQ | ID | NO: | 35: | TR96 | light | chain | CDR1 |
| SEQ | ID | NO: | 36: | TR96 | light | chain | CDR2 |
| SEQ | ID | NO: | 37: | TR96 | light | chain | CDR3 |
| SEQ | ID | NO: | 38: | TR109 | light | chain | CDR1 |
| SEQ | ID | NO: | 39: | TR109 | light | chain | CDR2 |
| SEQ | ID | NO: | 40: | TR109 | light | chain | CDR3 |

## Claims

1. An epitope region-bridging biparatopic antibody or an antigen binding fragment or functional equivalent thereof, comprising sequences of a heavy chain variable region and a light chain variable region derived from an antibody to a first epitope in an antigen that is activated by a target molecule binding thereto, and sequences of a heavy chain variable region and a light chain variable region derived from an antibody to a second epitope that is different from the first epitope, wherein a complex having a structure with antigen:antibody of (m × n):n is formed by binding to the antigen, wherein n is the same number that is 1 or greater, and m is a maximum value of the number of antigen binding domains sharing a paratope.

2. The epitope region-bridging biparatopic antibody or an antigen binding fragment or functional equivalent thereof of claim 1, wherein the first epitope belongs to a first epitope region selected from a plurality of epitope regions in the antigen, and the second epitope belongs to a second epitope region that is different from the first epitope region.

3. The epitope region-bridging biparatopic antibody or an antigen binding fragment or functional equivalent thereof of claim 1 or 2, wherein m is 1.

4. The epitope region-bridging biparatopic antibody or an antigen binding fragment or functional equivalent thereof of any one of claims 1 to 3, wherein a complex having an intramolecular bridged structure with antigen: antibody of 1:1 is formed by binding to the antigen.

5. The epitope region-bridging biparatopic antibody or an antigen binding fragment or functional equivalent thereof of any one of claims 1 to 3, wherein a complex having an intermolecular bridged structure with antigen: antibody of n:n (where n is the same number that is 2 or greater) is formed by binding to the antigen.

6. The epitope region-bridging biparatopic antibody or an antigen binding fragment or functional equivalent thereof of any one of claims 1 to 4, having antigen specificity to the first epitope and the second epitope that are present on the same side from each other on the antigen when binding to the antigen.

7. The epitope region-bridging biparatopic antibody or an antigen binding fragment or functional equivalent thereof of any one of claims 1 to 3 and 5, having antigen specificity to the first epitope and the second epitope that are present on different sides from each other on the antigen when binding to the antigen.

8. The epitope region-bridging biparatopic antibody or an antigen binding fragment or functional equivalent thereof of any one of claims 1 to 4 and 6, having antigen specificity to the first epitope and the second epitope that are present on a side that binds to the target molecule on the antigen when binding to the antigen.

9. The epitope region-bridging biparatopic antibody or an antigen binding fragment or functional equivalent thereof of any one of claims 1 to 3, 5, and 7, having antigen specificity to the first epitope that is present on a side that binds to the target molecule on the antigen and the second epitope that is present on a side that does not bind to the target molecule on the antigen when binding to the antigen.

10. The epitope region-bridging biparatopic antibody or an antigen binding fragment or functional equivalent thereof of any one of claims 1 to 4, 6, and 8, wherein antagonistic activity is enhanced relative to a naturally-occurring antibody.

11. The epitope region-bridging biparatopic antibody or an antigen binding fragment or functional equivalent thereof of any one of claims 1 to 4, 6, 8, and 10, wherein agonistic activity is suppressed relative to a naturally-occurring antibody.

12. The epitope region-bridging biparatopic antibody or an antigen binding fragment or functional equivalent thereof of any one of claims 1 to 11, wherein the antigen is a membrane protein.

13. The epitope region-bridging biparatopic antibody or an antigen binding fragment or functional equivalent thereof of any one of claims 1 to 12, wherein the antigen is a membrane protein belonging to a TNF receptor superfamily.

14. The epitope region-bridging biparatopic antibody or an antigen binding fragment or functional equivalent thereof of any one of claims 1 to 13, wherein the antigen is TNFR2.

15. An epitope region-bridging biparatopic antibody or an antigen binding fragment or functional equivalent thereof to TNFR2, comprising:
(a) a heavy chain comprising CDR1, CDR2, and CDR3 of each of two heavy chain variable regions selected from the group consisting of SEQ ID NO: 1 (TR45 heavy chain), SEQ ID NO: 2 (TR92 heavy chain), SEQ ID NO: 3 (TR94 heavy chain), SEQ ID NO: 4 (TR96 heavy chain), and SEQ ID NO: 5 (TR109 heavy chain), or a functionally equivalent sequence thereof, or an amino acid sequence having sequence identity of at least 80% to said amino acid sequences; and
(b) a light chain comprising CDR1, CDR2, and CDR3 of each of two light chain variable regions selected from the group consisting of SEQ ID NO: 6 (TR45 light chain), SEQ ID NO: 7 (TR92 light chain), SEQ ID NO: 8 (TR94 light chain), SEQ ID NO: 9 (TR96 light chain), and SEQ ID NO: 10 (TR109 light chain), wherein the light chain variable regions are derived from the same antibody as the antibody heavy chain of (a), or a functionally equivalent sequence thereof, or an amino acid sequence having sequence identity of at least 80% to said amino acid sequences.

16. The epitope region-bridging biparatopic antibody or an antigen binding fragment or functional equivalent thereof of claim 15, comprising:
(a) a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences; and
(b) a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences.

17. The epitope region-bridging biparatopic antibody or an antigen binding fragment or functional equivalent thereof of claim 15, which is:
(i) an antibody comprising
(a1) a heavy chain comprising CDR1, CDR2, and CDR3 of the amino acid sequence set forth in SEQ ID NO: 1 (TR45 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and
(b1) a light chain comprising CDR1, CDR2, and CDR3 of the amino acid sequence set forth in SEQ ID NO: 6 (TR45 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences;
(ii) an antibody comprising
(a2) a heavy chain comprising CDR1, CDR2, and CDR3 of the amino acid sequence set forth in SEQ ID NO: 1 (TR45 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and
(b2) a light chain comprising CDR1, CDR2, and CDR3 of the amino acid sequence set forth in SEQ ID NO: 6 (TR45 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences;
(iii) an antibody comprising
(a3) a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and
(b3) a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences;
(iv) an antibody comprising
(a4) a heavy chain comprising CDR1, CDR2, and CDR3 of the amino acid sequence set forth in SEQ ID NO: 4 (TR96 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and
(b4) a light chain comprising CDR1, CDR2, and CDR3 of the amino acid sequence set forth in SEQ ID NO: 9 (TR96 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences;
(v) an antibody comprising
(a5) a heavy chain comprising CDR1, CDR2, and CDR3 of the amino acid sequence set forth in SEQ ID NO: 5 (TR109 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and
(b5) a light chain comprising CDR1, CDR2, and CDR3 of the amino acid sequence set forth in SEQ ID NO: 10 (TR109 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences; or
(vi) an antibody comprising
(a6) a heavy chain comprising CDR1, CDR2, and CDR3 of the amino acid sequence set forth in SEQ ID NO: 3 (TR94 heavy chain), or a functionally equivalent sequence thereof, or an amino acid sequence having sequence identity of at least 80% to said amino acid sequences, and a heavy chain comprising CDR1, CDR2, and CDR3 of the amino acid sequence set forth in SEQ ID NO: 2 (TR92 heavy chain), or a functionally equivalent sequence thereof, or an amino acid sequence having sequence identity of at least 80% to said amino acid sequences, and
(b6) a light chain comprising CDR1, CDR2, and CDR3 of the amino acid sequence set forth in SEQ ID NO: 8 (TR94 light chain), or a functionally equivalent sequence thereof, or an amino acid sequence having sequence identity of at least 80% to said amino acid sequences, and a light chain comprising CDR1, CDR2, and CDR3 of the amino acid sequence set forth in SEQ ID NO: 7 (TR92 light chain), or a functionally equivalent sequence thereof, or an amino acid sequence having sequence identity of at least 80% to said amino acid sequences.

18. A method of manufacturing an epitope region-bridging biparatopic antibody or an antigen binding fragment or functional equivalent thereof, comprising the steps of:
(a) providing a population of antibodies to an antigen as an original antibody panel, wherein a number n of antibodies contained in the population of antibodies is a number N of target antibodies to the antigen or greater;
(b) obtaining binding data for antibodies contained in the original antibody panel;
(c) clustering the original antibody panel based on the binding data;
(d) excluding one or more antibodies from the original antibody panel as needed to generate one or more partial panels and clustering each of the one or more partial panels based on the binding data;
(e) calculating a number e of epitope groups of the original antibody panel and the one or more partial panels, and if there is an original antibody panel or partial panel satisfying e ≥ number E of target epitope groups related to the antigen, selecting the original antibody panel or partial panel satisfying e ≥ E as an epitope normalized antibody panel, but if there is no original antibody panel or partial panel satisfying e ≥ E, adding a new antibody to the original antibody panel or the partial panel to create a new population of antibodies, and repeating (a) to (d);
(f) selecting two epitopes from a plurality of epitopes included in the obtained epitope normalized antibody panel; and
(g) linking Fab regions of an antibody that bind to the two epitopes.

19. The method of claim 18, wherein step (f) selects the two epitopes so that positions of the two epitopes are on the same side on the antigen.

20. The method of claim 18, wherein step (f) selects the two epitopes so that positions of the two epitopes are on opposite sides on the antigen.
